# EUROPEAN PATENT APPLICATION

(11) **EP 2 108 705 A1**
(43) Date of publication of application: **14.10.2009**
(21) Application number: 08006965.1
(22) Date of filing: 08.04.2008
(51) Int. Cl.: C12Q 1/68

(54) **Method for analysing the epigenetic status of the HtrA 1 gene in a biological sample**

(71) Applicant: Universität Duisburg-Essen, 45141 Essen (DE)
(72) Inventor: Ehrmann, Michael, Prof. Dr., 45133 Essen (DE); Irle, Inga, 45239 Essen (DE); Schmidt, Nina, 44225 Dortmund (DE); Tennstädt, Annette, 50670 Köln (DE)
(74) Representative: Roth, Carla

(57) **Abstract**

The invention relates to a method for analysing a biological sample, wherein the epigenetic status of at least one section of the HtrA 1 gene is analysed. Furthermore, diagnostic kits as well as a screening method for identifying a molecule which inhibits the binding of an epigenetic factor to at least one section of the HtrA1 gene are provided.

## Description

The present invention pertains to methods for analysing a biological sample, in particular tumour tissue, by determining the epigenetic status of at least one section of the HtrA1 gene. Respective methods are useful in tumour/cancer diagnostics.

Even though cancer is perhaps the most complicated and diverse disease known, all cancers share common events. Hanahan and Weinberg postulated that 6 alterations are required to promote malignancy: self-sufficiency in growth signals, insensitivity to growth-inhibitory (antigrowth) signals, evasion of programmed cell death (apoptosis), limitless replicative potential, sustained angiogenesis, and tissue invasion and metastasis (Hanahan et al. Cell 100:57-70 (2000)). These events require illegitimate gene expression. For example, tumour suppressor genes or oncogenes are mutated or transcribed at the wrong time or at unphysiological levels. In addition, more than one mutational event is required before a normal cell is converted into a cancerous cell that lacks the normal control of cell growth and cell-cell interactions. These mutations and their phenotypic consequences are possible if the normal DNA repair systems are not functioning properly and if the cycle checkpoints are inactivated.

The most well-known cell cycle checkpoint factor is the tumour suppressor p53 that puts a halt on proliferation when DNA damage occurs (Harris et al. Oncogene 24:2899-2908. (2005); (Aylon et al. Cell 130:597-600 (2007)). If the damage is not repaired cells are undergoing apoptosis. This control mechanism is inactivated in most cancers allowing these cells to escape the normal control of proliferation and homeostasis. p53 is regulated by MDM2 and 4. MDM2 is a ubiquitin ligase. Under non-stress conditions, ubiquitinylated p53 is degraded by the proteasome. MDM2 is known to be proteolysed following self-ubiquitinylation. MDM4 is similar to MDM2 but does not function as an ubiquitin ligase. MDM4 is believed to interact with p53 and to regulate its ability to act as a transcription factor. In addition to p53, many other cellular factors have been identified that are involved in DNA repair and cell cycle control tolerating genome instability and polyploidy. Polyploidy is considered to cause genetic instability, which is one way to anoiploidy and cancer (Ganem et al. Curr Opin Genet Dev 17:157-62 (2007)). Polyploidy can be caused by defects in cytokinesis or chromosome segregation (e.g. when large amounts of chromatin occupy the site of division). It can also be caused by a spindle assembly checkpoint arrest from which cells can recover and re-enter G1 phase as tetraploids (Brito et al. Curr Biol 16:1194-1200 (2006)).

For example, p53 activates another tumour suppressor gene p21 that halts cell cycle in case of polyploidy. Normally, p21 blocks entry into S phase when cells are polyploid. This depends on active p53 and p38 MAP kinase. Polyploidy can be synthetically induced by application of a natural product, cytochalasin B. Cytochalasin B causes actin problems and polyploidy. Interestingly, large amounts (10 µg/ml) of Cytochalasin B cause polyploidy and cell cycle arrest via p21 as cell cycle arrest in not observed in p21 null cells. Low doses (0.5 µg/ml) also cause polyploidy but no cell cycle arrest suggesting that other factors (stress) are also required (Ganem et al. Cell 131:437-40 (2007)).

Another important regulatory element of cancer development is the epigenetic control of gene expression (Bestor Nature 393:311-2 (1998); Bird et al. Cell 99:451-454 (1999)). Epigenetics is defined as a hereditable change in gene expression not involving a change in DNA sequence but rather DNA and histone modifications. DNA methylation occurs mainly in so called CpG islands that are short regions of DNA in which the frequency of the CG sequence is higher than in other regions. CpG islands are often located around the promoters of housekeeping genes or other genes frequently expressed in a cell.

Histone modifications including acetylation, methylation, phosphorylation and sumoylation are important in transcriptional regulation and many are stably maintained during cell division. Histone tails are normally positively charged due to amine groups present on their lysine and arginine amino acids. These positive charges help the histone tails to interact with and bind to the negatively charged phosphate groups on the DNA backbone. Acetylation, which occurs normally in a cell, neutralizes the positive charges on the histone by changing amines into amides and decreases the ability of the histones to bind to DNA.

Acetylation of histones (H) mainly H₃ and H₄ is done at lysine 9 and 14 and lysine 5, 8, 12, 16, respectively, by histone acetyltransferases (HATs). They transfer the acetyl moiety of coenzyme A to the lysine residue which leads to neutralizing the positive charge of lysine. Histone acetylation loosens chromatin packaging and correlates with transcriptional activation as the DNA is less densely packed in the chromatin. Histone Deacetylase (HDAC) removes those acetyl groups, restoring positive charges to the histone tails and encouraging high-affinity binding between the histones and DNA backbone. Histone Deacetelyases are a class of enzymes that remove acetyl groups from an ε-N-acetyl lysine amino acid on a histone. Its action is opposite to that of histone acetyltransferase. Deacetylation removes acetyl groups from histone tails, causing the DNA to wrap more tightly around the histones and interfering with the transcription of genes by blocking access by transcription factors. The overall result of histone deacetylation is a global (non specific) reduction in gene expression. Thus, Histone Deacetylases (HDACs) are normally associated with repression of transcription.

Epigenetic alterations are found in almost all types of cancers. Hypermethylation of promoter CpG islands associated with tumour suppressor genes is causing decreased expression levels/silencing. In addition, the observed global hypomethylation in cancer is connected with chromosome instability, activation of viruses and activation of proto-oncogenes (Ting et al. Genes Dev 20:3215-31 (2006)). Changes in chromatin structure include known histone modifications, however, little is known about histone acetylation except that cancer cells exhibit a loss of monoacetylated and trimethylated forms of histone H4. Interestingly, these changes appear early and accumulate during the tumourigenic process, indicating that they might be relevant steps in the transformation process. During the past few years, genes with key roles in cancer biology, such as the genes encoding the cell-cycle inhibitors p16, p21 and the DNA-repair genes MLH1 and BRCA1, have been shown to undergo methylation-associated silencing in cancer cells.

Hence, a growing number of tumour-suppressor and other cancer-related genes have been demonstrated to be silenced by aberrant promoter methylation. De novo Methylation of CpG islands in the promoter regions of tumour suppressor genes may lead to transcriptional silencing through a complex process involving histone deacetylation and chromatin condensation, and thus represents a tumourigenic event that is functionally equivalent to genetic changes, such as mutation and deletion. The profile of promoter hypermethylation at genes differs for each cancer type, providing a tumour-type and gene-specific profile. An aberrant methylation of certain genes reflects the very specific involvement in selected tumour or groups of tumour-types. Aberrant DNA methylation is also involved in drug resistance of tumours. Gene silencing effects disease progression, resistance and clinical outcome following therapy, and a number of recent studies suggest a direct role for epigenetic inactivation of genes in determining tumour chemosensitivity. Numerous genetic and epigenetic changes in cancer cells may contribute to inactivate the function of oncosuppressor genes thus contributing to select a drug resistance phenotype.

Intrinsic and acquired drug resistances remain the most unpredictable factor affecting chemotherapy. DNA hypermethylation has been found to be associated with drug resistance acquired during cancer chemotherapy and therefore, re-expression of methylation-silenced genes results in increased sensitivity to existing chemotherapy. Therefore, a number of compounds that inhibit DNA methylation (e.g. Decitabine) or histone deacetylation (e.g. SAHA) are in clinical trials to evaluate their potential as drug either alone or in combination with established drugs (Gallinari et al. Cell Res. 17:195-211 (2007); Gore et al. Cancer Res. 66:6361-9 (2006)).

As a consequence of the above, the detection of epigenetically controlled genes implicated in tumour/cancer biology may serve as important biomarkers for early detection of cancer, for cancer risk assessment, for etiopathology prediction and for predicting the response to therapy. Since epigenetic changes such as methylation often appear early in disease, detection of hypermethylated genes could identify tissues derived from a subject with increased risk of cancer. Furthermore, the reversible nature of methylation offers a potential to revert aspects of the cancer phenotype with an appropriate therapy. Furthermore, determining or analysing respective epigenetically controlled biomarkers may improve the possibility to find the appropriate therapy and to provide a prognosis regarding cancer development.

It is thus the object of the present invention to identify new cancer markers that are epigenetically controlled. It is also an object of the present invention to provide suitable methods and kits for analyzing biological samples, in particular tumour samples, for the presence of respective biological markers.

According to a first embodiment of the present invention, a method for analyzing a biological sample is provided, wherein the epigenetic status of at least one section of the HtrA1 gene is analysed/determined. It was surprisingly found, that the epigenetic status of the HtrA1 gene, and in particular of the HtrA1 promoter plays an important role in the development of cancer/tumours. Thus, the analysis and in particular the determination of the epigenetic status of the HtrA1 gene and in particular the HtrA1 promoter provides an important biomarker for the analysis and diagnosis of cancer.

The term "HtrA1 gene" refers to a polynucleotide encoding HtrA1, comprising regulatory sequences such as the promoter and/or enhancer and coding and optionally non-coding sequences.

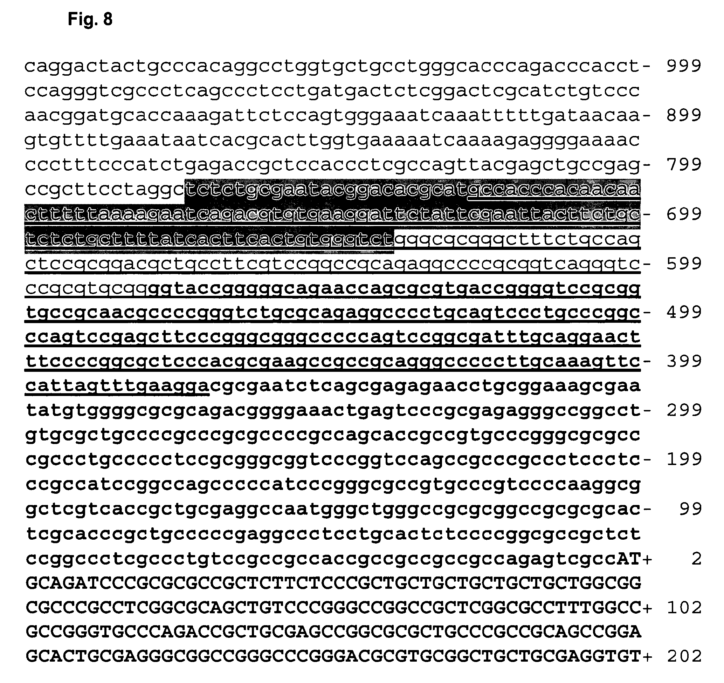

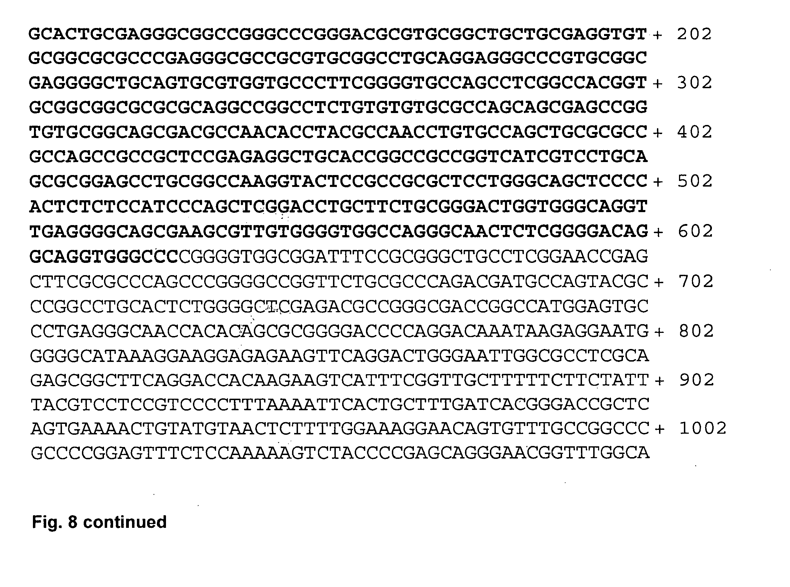

The term "HtrA1 promoter" refers to a regulatory region of the HtrA1 gene, which is located upstream of the coding sequences of the HtrA1 gene. A promoter provides a control point for regulated gene transcription. The promoter contains specific DNA sequences, response elements, that are recognized by proteins such as transcription factors or other regulatory factors. These factors bind to the promoter sequence of the HtrA1 gene. In particular, the term "HtrA1 promoter" refers to a sequence as shown in Fig. 8 or a portion/section thereof.

It was surprisingly shown by the inventors, that the expression of HtrA1 is epigenetically controlled in cancer/tumour cells, but not in the analysed non-cancer cells. The epigenetic status of the HtrA1 gene may thus serve as an important indicator for cancer/tumour tissue. This fact was so far unknown in the prior art. Thereby an important biomarker and risk factor was identified by the inventors, which is very useful in order to characterise and analyse the cancer/tumour. A respective characterisation also provides aid to the physician regarding the choice of the appropriate therapy and gives information about potential drug resistances. Furthermore, it provides an important indicator for the disease progression (e.g. the metastasis risk). Specifically, if the DNA of the HtrA1 promoter is found to be methylated and/or the histones interacting with the DNA are not posttranslationally modified (for example deacetylated), HtrA1 expression is silenced in the tumour context. The loss of HtrA1 expression is correlated with metastatic behaviour of tumours and resistance to commonly used chemotherapeutica such as taxol and platins. Therefore, a quantitative analysis of the epigentic status of the HtrA1 promoter allows a direct correlation with patient fate.

The HtrA (high temperature requirement) family represents a rather new class of oligomeric serine proteases, the activity of which is regulated by reversible zymogen activation. The defining feature of the over 180 family members is the combination of a catalytic domain with one or more C-terminal PDZ domains. PDZ domains are protein modules that mediate specific protein-protein interactions and bind preferentially to the C-terminal 3-4 residues of the target protein. Prokaryotic HtrAs have been attributed to the tolerance against various folding stresses as well as to pathogenicity. The four human homologues are believed to be involved in arthritis, cell growth, unfolded protein response, cancer, ageing, placental development and function, Parkinson, in metabolism of amyloid precursor protein and age-related macular degeneration (Abou-Sleiman et al., Nat Rev Neurosci 7:207-219 (2006); Clausen et al., Mol Cell 10, 443-455 (2002); Grau et al., Proc. Natl. Acad. Sci. USA 102:6021-6026 (2005); Nie et al., Placenta 27:491-501(2006); Dewan et al., Science 314:989-992 (2006); Yang et al., Science 314:992-993 (2006)).

The HtrA1 gene (PRSS11) was initially identified as being expressed in human fibroblasts but not after transformation with SV40 (Zumbrunn et al. FEBS Lett 398:187-92 (1996)). Recent studies indicate that PRSS11 mRNA is either absent or significantly down regulated in ovarian cancer (Shridhar et al., Cancer Res 62: 262-270 (2002); Chien et al. Oncogene 23:1636-44 (2004)), leukaemia, Burkitt's lymphoma, melanomas and endometrial cancer (Baldi et al., Oncogene 21:6684-6688 (2002); Bowden et al. Gynecol Oncol. 103:253-60 (2006)). In addition, overexpression of HtrA1 inhibited proliferation *in vitro* and tumour growth *in vivo* (Baldi et al., Oncogene 21:6684-6688 (2002)). These results suggest a tumour suppressor function. A tumour suppressor phenotype of a protease is interesting as so far this function was mainly attributed to protease inhibitors. HtrA1 is mainly secreted but a subfraction is also localised in the cytosol (Grau et al., J Biol Chem 281:6124-6129 (2006)).

Secreted HtrA1 is likely to be involved in the degradation of extracellular matrix proteins and APP fragments (Grau et al., Proc Natl Acad Sci USA 102:6021-6026 (2005)).

The serine protease HtrA1 has been previously implicated in tumour biology. It was shown that its expression is downregulated in tumours and forcing its re-expression in cell culture interfered with cell proliferation (Chien et al. Oncogene 23:1636-44 (2004)). Interestingly, the levels of HtrA1 expression correlate with sensitivity to commonly applied cancer drugs cisplatin and taxol. Here, low levels of HtrA1 correlated with a poor response to drug treatment while higher levels of HtrA1 correlated with good responses (Chien et al. J Clin Invest 116:1994-2004 (2006)). However, so far it was unknown that the HtrA1 gene is epigenetically controlled in cancer/tumour cells.

Preferably, the epigenetic status of at least one section of the HtrA 1 promoter is analysed.

According to one embodiment, the epigenetic status is analysed by analyzing the
- DNA methylation status,
- the histone acetylation status, and/or
- the expression and/or binding of an epigenetic factor.

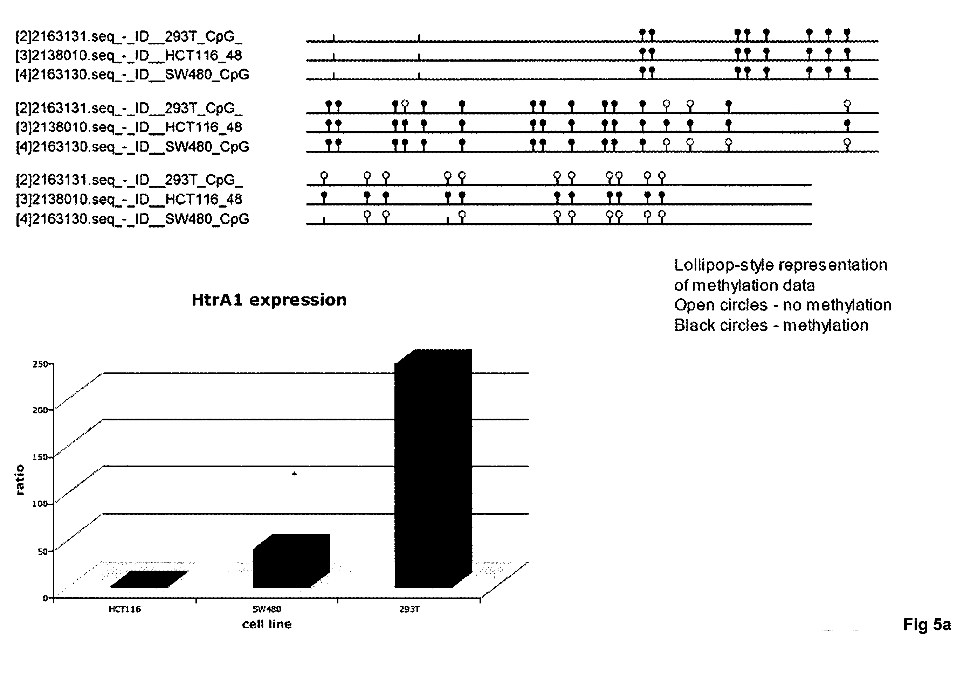

When analyzing the methylation status of the HtrA1 gene, it was found that in particular the CpG island of the HtrA1 promoter plays an important role in the epigenetic regulation of the HtrA1 gene. Thus, according to one embodiment, the methylation status of at least one section of the HtrA1 promoter and in particular a CpG island in at least one section of the HtrA1 promoter is analysed/determined. Also further sections of the HtrA1 gene may be analysed in addition to the at least one section of the HtrA1 promoter. Preferably, that at least one section of the HtrA1 promoter comprises at least part of the sequence of bp -762 to -383 upstream of the ATG start codon of the HtrA1 gene (as shown in Fig. 8). Also Fig. 5a indicates suitable sections of the HtrA1 promoter.

Hence, preferably, said at least one section of the HtrA1 promoter comprises or consists of at least a portion of a sequence selected from the group consisting of
a. the sequence shown in Fig. 8;
b. the sequence 
gccacccacaacaacttttaaaagaatcagacgtgtgaaggattctatt
cgaattacttctgctctctgcttttatcacttcactgtgggtctgggcgc
gggctttctgccagctccgcggacgctgccttcgtccggccgcagaggcc
ccgcggtcagggtcccgcgtgcggggtaccgggggcagaaccagcgcgtg
accggggtccgcggtgccgcaacgccccgggtctgcgcagaggcccctgc
 
agtccctgcccggcccagtccgagcttcccgggcgggcccccagtccggc
gatttgcaggaactttccccggcgctcccacgcgaagccgccgcagggcc
cccttgcaaagttccattagtttgaagga;
c. the sequence 
cgcgaatctcagcgagagaacctgcggaaagcgaatatgtggggcgcgca
gacggggaaactgagtcccgcgagagggccggcctgtgcgctgccccgcc
cgcgccccgccagcaccgccgtgcccgggcgcgcccgccctgcccccctcc
gcgggcggtcccggtccagccgcccgccctccctcccgccatccggccag
cccccatcccgggcgccgtgcccgtccccaaggcggctcgtcaccgctgc
gaggccaatgggctgggccgcgcggccgcgcactcgcacccgctgccc
ccgaggccctcctgcactctccccggcgccgctctccggccctcgccctg
tccgccgccaccgccgccgccgccagagtcgcc;
d. the sequence 
atgcagatcccgcgcgccgctcttctcccgctgctgctgctgctggc
ggcgcccgcctcggcgcagctgtcccgggccggccgctcggcgcctttgg
ccgccgggtgcccagaccgctgcgagccggcgcgctgcccgccgcagccg
gagcactgcgagggcggccgggcccgggacgcgtgcggctgctgcgaggt
gtgcggcgcgcccgagggcgccgcgtgcggcctgcaggagggcccgtgcg
gcgaggggctgcagtgcgtggtgcccttcggggtgccagcctcggccacg
gtgcggcggcgcgcgcaggccggcctctgtgtgtgcgccagcagcgagcc
ggtgtgcggcagcgacgccaacacctacgccaacctgtgccagctgcgcg
ccgccagccgccgctccgagaggctgcaccggccgccggtcatcgtcctg
cagcgcggagcctgcggccaaggtactccgccgcgctcctgggcagctcc
ccactctctccatcccagctcggacctgcttctgcgggactggtgggcag
gttgaggggcagcgaagcgttgtggggtggccagggcaactctcggggac
aggcaggtgggccc
 and/or a functional variant thereof. A functional variant of the HtrA1 promoter may, e.g. comprise allelic variations of the shown sequences. Said portion/section of the shown sequences comprises preferably at least 10, 50, 70 and/or 100 bp.

Preferably, the methylation status of the HtrA1 gene and in particular the HtrA1 promoter is determined quantitatively. This, as the level of methylation provides insight, regarding the expression level of the HtrA1 gene. The expression level of the HtrA1 gene can be confirmed by conventional methods, such as ELISA, Northern Blots and quantitative RT-PCR. To obtain the information about the methylation status of the HtrA1 gene is an important information in addition to the knowledge of the overall HtrA1 expression rate, as it provides information regarding the precise cause for a downregulation of the HtrA1 gene and its nature, e.g. that it is reversible if it is due to DNA methylation.

There are several methods known in the prior art in order to determine the methylation status of a polynucleotide sequence. According to one embodiment, the methylation status is detected by at least of the following methods:
(i) restriction enzyme digestion methylation detection;
(ii) bisulphate-based methylation detection;
(iii) mass-spectrometry analysis;
(iv) sequence analysis;
(vi) methylation density assay;
(vii) immunoprecipitation of methylated sequences; and/or
(viii) methylation-specific PCR.

Accordingly, several approaches for determining DNA methylation are known and available in the art including restriction enzyme digestion-based methylation detection and bisulphate-based methylation detection. Several such approaches are e.g. disclosed in Ahrendt (1999) J. Natl. Cancer Inst. 91:332-9; Belinsky (1998) Proc. Natl. Acad. Sci. USA 95:11891-96; Clark (1994) Nucleic Acids Res. 22:2990-7; Herman (1996) Proc. Natl. Acad. Sci. USA 93:9821-26; Xiong and Laird (1997) Nuc. Acids Res. 25:2532-2534).

Restriction enzyme digestion methylation detection assay is based on the inability of some restriction enzymes to cut methylated DNA. Typically used are the enzyme pairs Hpall-Mspl including the recognition motif CCGG, and Smal-Xmal with a less frequent recognition motif, CCCGGG. Thus, for example, Hpall is unable to cut DNA when the internal cytosine in methylated, rendering Hpall-Mspl a valuable tool for rapid methylation analysis. The method is usually performed in conjunction with a Southern blot analysis. Since digestion by methylation sensitive enzymes (e.g., Hpall) is often partial, a complementary analysis with McrBC or other enzymes which digest only methylated CpG sites is preferable (Yamada et al. Genome Research 14 247-266 2004) to detect various methylation patterns.

Bisulphate-based methylation genomic sequencing is described in Clark et al., (1994) supra, and is capable of detecting every methylated cytosine on both strands of any target sequence. In this method, sodium bisulphite is used to convert cytosine residues to uracil residues in single- stranded DNA, under conditions whereby 5-methylcytosine remains nonreactive. The converted DNA is amplified with specific primers and sequenced. All the cytosine residues remaining in the sequence represent previously methylated cytosines in the genome. This method utilizes defined procedures that maximize the efficiency of denaturation, bisulphite conversion and amplification, to permit methylation mapping of single genes from small amounts of genomic DNA.

Methylation-specific PCR (MSP) is the most widely used assay for the sensitive detection of methylation. Briefly, prior to amplification, the DNA is treated with sodium bisulphite to convert all unmethylated cytosines to uracils. The bisulphite reaction effectively converts methylation information into sequence difference. The DNA is amplified using primers that match one particular methylation state of the DNA, such as that in which DNA is methylated at all CpGs. If this methylation state is present in the DNA sample, the generated PCR product can be visualized on a gel. It will be appreciated, though, that the method specific priming requires all CpG in the primer binding sites to be co-methylated. Thus, when there is comethylation, an amplified product is observed on the gel. When one or more of the CpGs is unmethylated, there is no product. Therefore, the method does not allow discrimination between partial levels of methylation and complete lack of methylation (see U.S. Pat. No. 5,786,146; Herman et al., Proc. Natl. Acad. Sci. USA 93: 9821-9826 (1996)). Real-time fluorescent MSP (MethyLight) is based on real time PCR employing fluorescent probes in conjunction with MSP and allows for a homogeneous reaction which is of higher throughput. If the probe does not contain CpGs, the reaction is essentially a quantitative version of MSP. However, the fluorescent probe is typically designed to anneal to a site containing one or more CpGs, and this third oligonucleotide increases the specificity of the assay for completely methylated target strands. Because the detection of the amplification occurs in real time, there is no need for a secondary electrophoresis step. Since there is no post PCR manipulation of the sample, the risk of contamination is reduced. The MethyLight probe can be of any format including but not limited to a Taqman probe or a LightCycler hybridization probe pair and if multiple reporter dyes are used, several probes can be performed simultaneously (Eads (1999) Cancer Res. 59:2302- 2306; Eads (2000) Nucleic Acids Res. 28:E32; Lo (1999) Cancer Res. 59:3899-390). There are also commercially available kits for determining the methylation status.

Methylation density assay is a quantitative method for rapidly assessing the CpG methylation density of a DNA region as previously described by Galm et al. (2002) Genome Res. 12, 153-7. Basically, after bisulfite modification of genomic DNA, the region of interest is PCR amplified with nested primers. PCR products are purified and DNA amount is determined. A predetermined amount of DNA is incubated with ³H-SAM (TRK581 Bioscience, Amersham) and Sssl methyltransferase (M0226, New England Biolabs Beverly, MA 01915-5599, USA) for methylation quantification. Once reactions are terminated products are purified from the in-vitro methylation mixture. 20 % of the eluant volume is counted in ³H counter. Normalizing radioactivity DNA of each sample is measured again and the count is normalized to the DNA amount.

Restriction analysis of bisulfite modified DNA is a quantitative technique also called COBRA (Xiong and Laird, 1997, Nuc. Acids Res. 25:2532-2534) which can be used to determine DNA methylation levels at specific gene loci in small amounts of genomic DNA. Respective methods are known and thus need no detailed description. Restriction enzyme digestion is used to reveal methylation-dependent sequence differences in PCR products of sodium bisulfite- treated DNA. Methylation levels in original DNA sample are represented by the relative amounts of digested and undigested PCR product in a linearly quantitative fashion across a wide spectrum of DNA methylation levels. COBRA thus combines the powerful features of ease of use, quantitative accuracy, and compatibility with paraffin sections.

Immunoprecipitation of methylated sequences can also be used to isolate sequence- specific methylated DNA fragments. The details are known in the prior art and are thus not described herein.

It will also be appreciated that a number of commercially available kits may also be used to detect the methylation status of the locus of the present invention. Typically, oligonucleotides for the bisulfite-based methylation detection methods described hereinabove are designed according to the technique selected (please also refer to the examples).

According to a further embodiment of the present invention, the acetylation status of the histones in particular bound to the HtrA1 gene is determined. As it is outlined above, a further important epigenetic pattern influencing the expression of genes can be the acetylation status of the histones. In case the histones are present in the deacetylated form, this is indicative for a tight DNA packaging and thus a low or even absent transcription of the HtrA1 gene. The knowledge of the acetylation status at the HtrA1 gene and in particular the HtrA1 promoter is important, as it provides insight in the nature/genetic reasons for the disease.

The acetylation status of the histones bound to the HtrA1 gene, and in particular to the HtrA1 promoter, may be performed by a sequence specific ChIP analysis.

Since HDACs mediate deacetylation of histones and the amount of HDAC1 (or HDAC2) bound to the HtrA1 promoter directly correlates with the activity of the HtrA1 promoter, the deacetylation of the HtrA1 promoter can be analysed by CHIP experiments by using antibodies against e.g. HDAC1 and subsequent amplification of appropriate HtrA1 promoter elements using the primers described in Example 5 or other suitable primers. Alternatively, ChIP experiments using antibodies for example against acetylated histones 3, 4 and acetylated lysine 9 of histone 3 can be performed. DNA fragments isolated from immunoprecipitated fractions are subsequently analyzed by semiquantitative or quantitative PCR using primers specific for the HtrA1 CpG island to be analysed.

A respective analysis of the acetylation status of the histones bound to the HtrA1 gene, and in particular to the HtrA1 promoter, can be performed as alternative or in addition to an analysis of the methylation status of the HtrA1 gene, and in particular of the methylation status of at least one section of the HtrA1 promoter as described above.

According to a further embodiment, the expression level of an epigenetic factor binding the methylated form of the HtrA1 gene and in particular the HtrA1 promoter is determined. Preferably, said epigenetic factor is a key modulator of the histone acetylation status. One example for a respective epigenetic factor, which binds and regulates the HtrA1 gene is MBD2. The expression level of the respective epigenetic factor can be determined for example by ELISA methods or by PCR methods, such as e.g. quantitative PCR. It was found by the inventors that if MBD2 is expressed in the sample and is in particularly expressed at a higher level, that the HtrA1 gene is downregulated. In addition, the downregulation of MBD2 results in a strong upregulation of HtrA1 promoter activity. The expression of MBD2 thus strongly correlates with the expression of HtrA1. Normally, epigenetic factors regulate many different genes. However, it was now surprisingly found that MBD2 only regulates a few genes, among which is the HtrA1 gene. This result correlates with in vivo studies where MBD2 null mice display a very weak phenotype while the MBD3 null genotype causes embryonic lethality.

MBD2 binds to the HtrA1 promoter in case the promoter is at least partially methylated. This was presently shown by CHIP experiments. Thus, the determination of the expression level of the epigenetic factor and in particular the expression of MBD2 can be performed as an alternative or in addition to determining the DNA methylation status of the HtrA1 gene, and in particular the HtrA1 promoter and/or as an alternative or in addition to determining the acetylation status of the histones at the HtrA1 gene, and in particular the HtrA1 promoter. MBD2 acts as a methylation-dependent transcriptional repressor. MBD2 excerpts different activities depending on its association with different partners. In particular, MBD2 recruits large NuRD/Mi-2 complexes to methylated DNA sequences. These complexes contain HDACs, histone methly transferases, histone-binding proteins such as RbAp46 and RbAp48, nucleosome remodelling enzyme Mi-2, metastasis-associated proteins MTA1 and MTA2 and other methyl binding domain proteins such as Mbd3. MBD2 may target deacetylase activity at methylated sites, thereby causing deacetylation of histones and thus further promoting suppression of the HtrA1 gene due to a more dense packaging of the chromatin.

The method of the present invention is in particular suitable to characterize tumour and cancer samples/tissues, in particular in order to alleviate the diagnosis and in order to provide suitable information regarding the nature of the tumour/cancer and its characteristics (e.g. the metastasis risk). Thereby, the therapy can be adapted and the physician gains valuable insight to the tumour ethiology.

Thus, the biological sample preferably comprises or consists of tumour/cancer cells. The biological sample comprising or consists of tumour/cancer cells can be isolated from the subject to be analysed/diagnosed.

According to one embodiment, the nucleic acids are isolated from tumour and/or cancer tissue, and the methylation status of at least one section of the HtrA 1 promoter is analysed. In order to perform the analysis of the epigenetic status, for example the DNA methylation status of the HtrA1 gene, nucleic acids can be isolated from the tumour/cancer tissue by using conventional methods, and the methylation status of at least one section of the HtrA1 promoter is determined, and/or the expression of an epigenetic factor is analyzed for example by RT-PCR and/or the acetylation status of the histones and the HtrA1 gene is determined.

Also provided is a method for analysing and/or diagnosing cancer and/or a tumour, which is **characterised in that** the method described above is performed.

Also provided is a diagnostic kit for performing a method according to the present invention. A respective diagnostic kit may include suitable buffers and reagents for performing the above described methods. The methods of the present invention are thus useful in tumour/cancer diagnosis.

A diagnostic kit for analysing the methylation status of at least one section of the HtrA 1 gene may comprises suitable primers for amplifying at least of portion of the sequence shown in Fig. 8. Preferably, the primers are designed such that they hybridise to at least one of the sequences selected from the group consisting of:
a. the sequence: 
gccacccacaacaactttttaaaagaatcagacgtgtgaaggattctatt
cgaattacttctgctctctgcttttatcacttcactgtgggtctgggcgc
gggctttctgccagctccgcggacgctgccttcgtccggccgcagaggcc
ccgcggtcagggtcccgcgtgcggggtaccgggggcagaaccagcgcgtg
accggggtccgcggtgccgcaacgccccgggtctgcgcagaggcccctgc
agtccctgcccggcccagtccgagcttcccgggcgggcccccagtccggc
gatttgcaggaactttccccggcgctcccacgcgaagccgccgcagggcc
cccttgcaaagttccattagtttgaagga;
b. the sequence: 
cgcgaatctcagcgagagaacctgcggaaagcgaatatgtggggcgcgca
gacggggaaactgagtcccgcgagagggccggcctgtgcgctgccccgcc
cgcgccccgccagcaccgccgtgcccgggcgcgcccgccctgcccccctcc
gcgggcggtcccggtccagccgcccgccctccctcccgccatccggccag
cccccatcccgggcgccgtgcccgtccccaaggcggctcgtcaccgctgc
gaggccaatgggctgggccgcgcggccgcgcgcactcgcacccgctgccc
ccgaggccctcctgcactctccccggcgccgctctccggccctcgccctg
tccgccgccaccgccgccgccgccagagtcgcc;
c. the sequence: 
atgcagatcccgcgcgccgctcttctcccgctgctgctgctgctggc
ggcgcccgcctcggcgcagctgtcccggccggccgctcggcgcctttgg
ccgccgggtgcccagaccgctgcgagccggcgcgctgcccgccgcagccg
gagcactgcgagggcggccgggcccgggacgcgtgcggctgctgcgaggt
gtgcggcgcgcccgagggcgccgcgtgcggcctgcaggagggcccgtgcg
gcgaggggctgcagtgcgtggtgcccttcggggtgccagcctcggccacg
gtgcggcggcgcgcgcaggccggcctctgtgtgtgcgccagcagcgagcc
ggtgtgcggcagcgacgccaacacctacgccaacctgtgccagctgcgcg
ccgccagccgccgctccgagaggctgcaccggccgccggtcatcgtcctg
cagcgcggagcctgcggccaaggtactccgccgcgctcctgggcagctcc
ccactctctccatcccagctcggacctgcttctgcgggactggtgggcag
gttgaggggcagcgaagcgttgtggggtggccagggcaactctcggggac
aggcaggtgggccc.
 Also provided is a screening method for identifying a molecule which inhibits the binding of an epigenetic factor to at least one section of the HtrA1 gene, comprising

a. contacting an inhibitor candidate molecule with a sample comprising at least the epigenetic factor and a polynucleotide molecule comprising or consisting of the section of the HtrA 1 gene the epigenetic factor binds to,
b. determining the degree of binding of the epigenetic factor to said polynucleotide.

Preferably, said polynucleotide is methylated.

According to one embodiment, said epigenetic factor is MBD2. According to one embodiment, the methylated polynucleotide comprises or consists of the following sequence or a variant thereof, which is still bound by MBD2 when methylated:

a.) tctctgcgaatacggacacgcatgccacccacaacaactttttaaaagaa
tcagacgtgtgaaggattctattcgaattacttctgctctctgcttttat
cacttcactgtgggtct

In addition, another region can be co-investigated and accordingly a polynucleotide can be used , which comprises or consists of the following sequence:

b.) ccccccttgcaaagttccattagtttgaaggacgcgaatctcagcgagaga
acctgcggaaagcgaatatgtggggcgcgcagacggggaaactgagtccc
gcgagag

The respective assay allows the identification of valuable therapeutic compounds that could be useful in cancer therapy by increasing the expression of the HtrA1 gene by inhibiting the binding of the epigenetic factor (which would inhibit or reduce HtrA1 gene expression) such as MBD2 to the HtrA1 gene.

### I. Brief description of the drawings

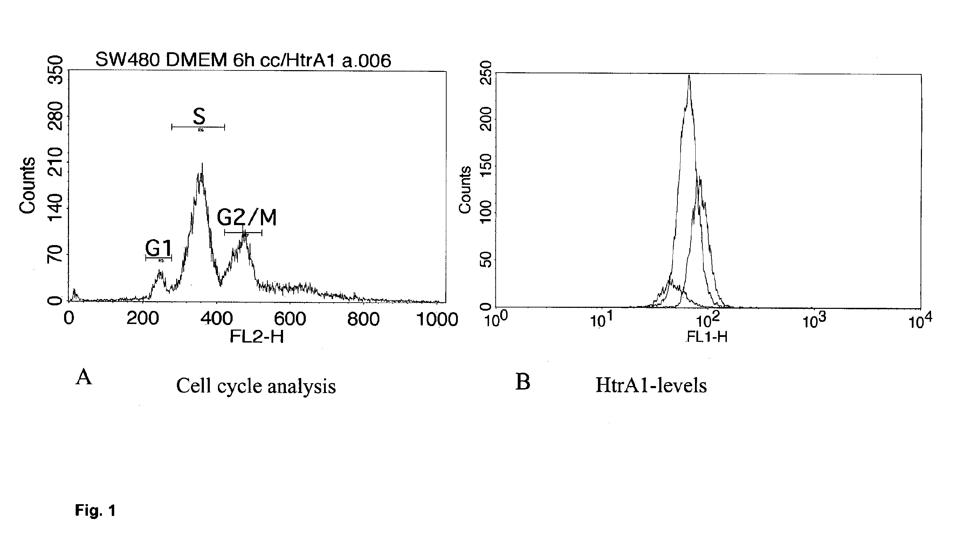

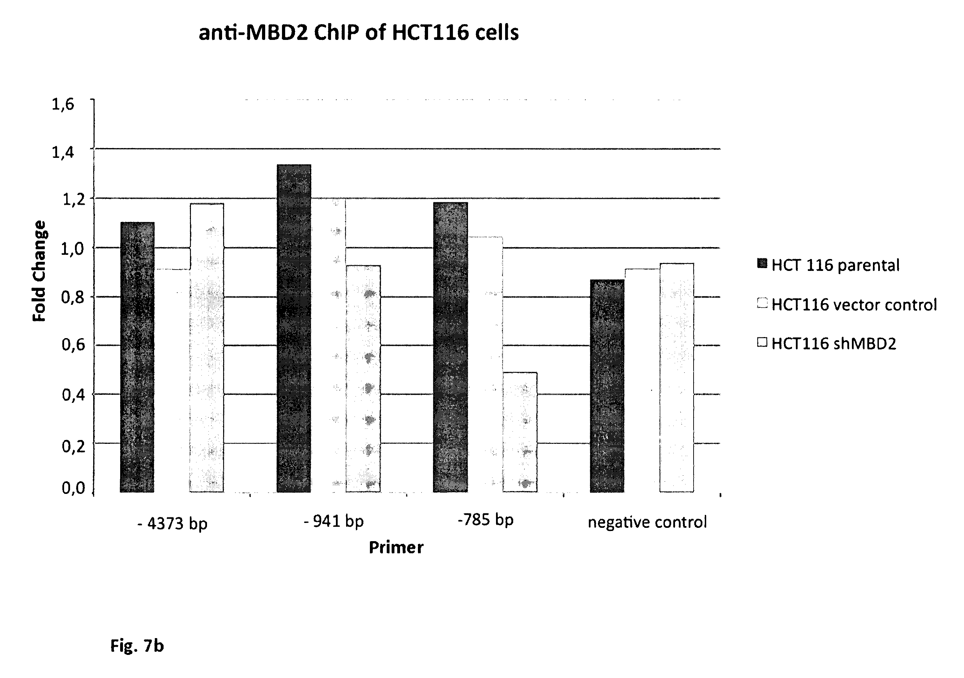

**Fig 1****: HtrA1 levels change during cell cycle** SW480 (colon cancer) cells were synchronized with 1.25 µg/ml Aphidicolin and analyzed for DNA content by propidium iodide staining and simultaneous for HtrA1 level by intracellular immunofluorescence staining.
   **A)** Analysis of DNA content and gating according to cell cycle phase (G1-phase (2n); S-phase; G2/M-phase (4n)).
   **B)** HtrA1 levels according to the gated cells in Fig 1A. Red is G1-phase, green is S-phase and blue is G2/M-phase. Cells in G2/M-phase have the highest level of HtrA1 and cells in G1-phase of the cell cycle have the lowest level of HtrA1.

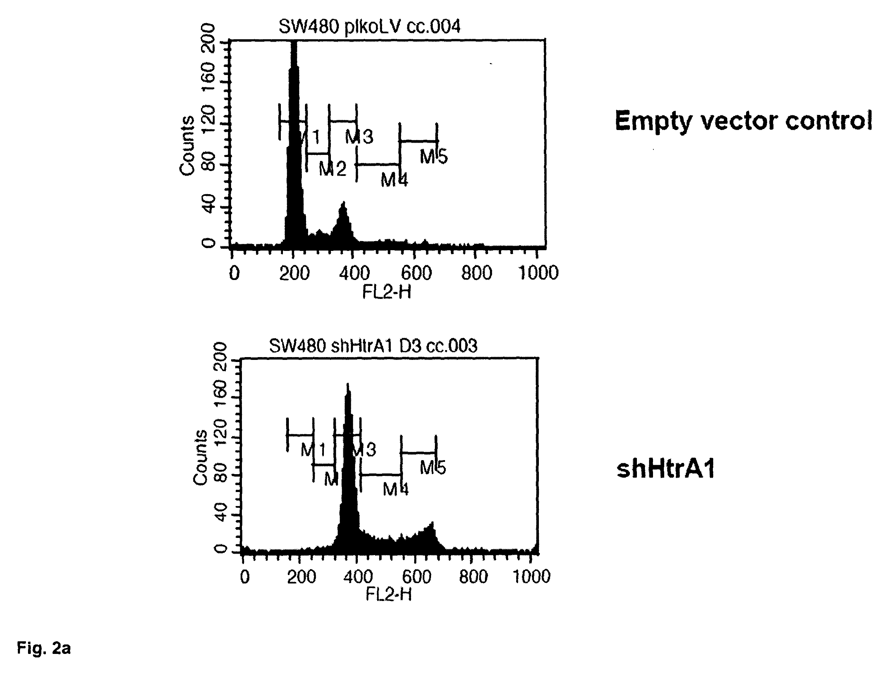

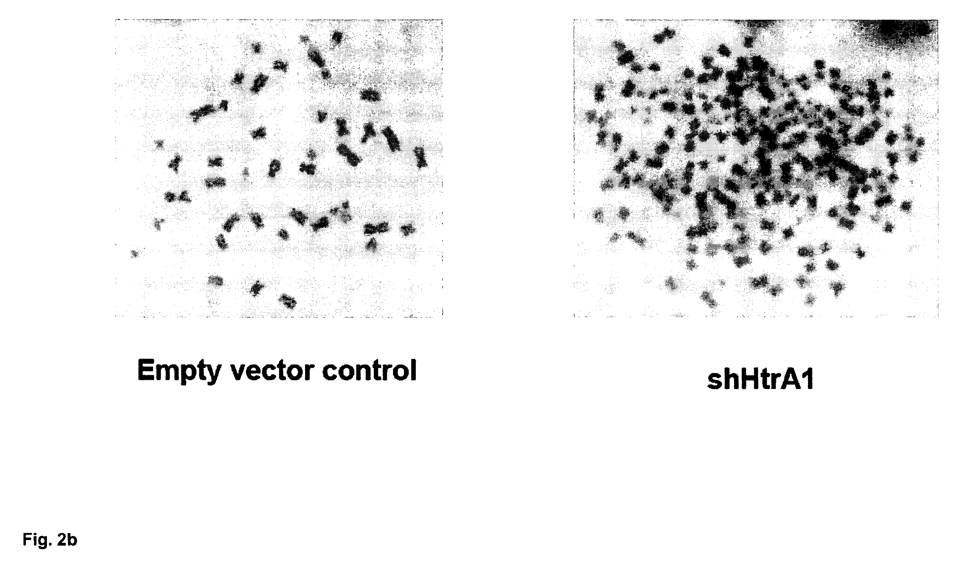

**Fig 2****: Polyploidy of SW480 cells**
   *A) Polyploidy of SW480 cells depleted for HtrA1*/*FACS analysis* SW480 (colon cancer) cells were transduced with a shRNA against HtrA1. After some passages cells were analyzed by propidium iodide staining for their DNA content. Gate M1 corresponds to a DNA content of 2n, gate M3 corresponds to a DNA content of 4n and gate M5 corresponds to a DNA content of 8n. SW480 cells expressing the shRNA against HtrA1 show a shift of the main peak from 2n to 4n and a shift of the smaller peak from 4n to 8n compared to SW480 cells containing the empty vector which suggests that cells with downregulated HtrA1 become polyploid.
   *B) Polyploidy of SW480 cells depleted for HtrA1*/*karyo-typing* Metaphase chromosomes of SW480 cell lines transduced with plko empty vector and plko vector containing a shRNA against HtrA1. SW480 cells transduced with an shRNA against HtrA1 show a higher grade of polyploidy compared to cells transduced with an empty vector control.
   Thus, a downregulation of HtrA1 correlates with the degree of polyploidy. As is outlined above, polyploidy is an indicator for cancer.

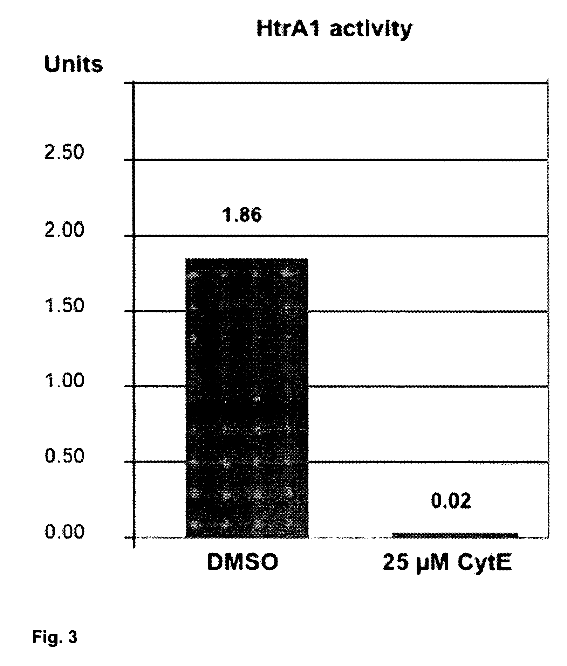

**Fig. 3****: Cytochalasin E is a potent HtrA1 inhibitor** HtrA1 activity was determined with a p-NA substrate. HtrA1 was incubated with either DMSO alone or 25 µM Cytochalasin E that was dissolved in DMSO. Reaction was measured spectrophometrically for 70 minutes at 37°C at 405 nm. It is known that Cytochalasins cause polyploidy when applied to cells in 1 µM concentrations.

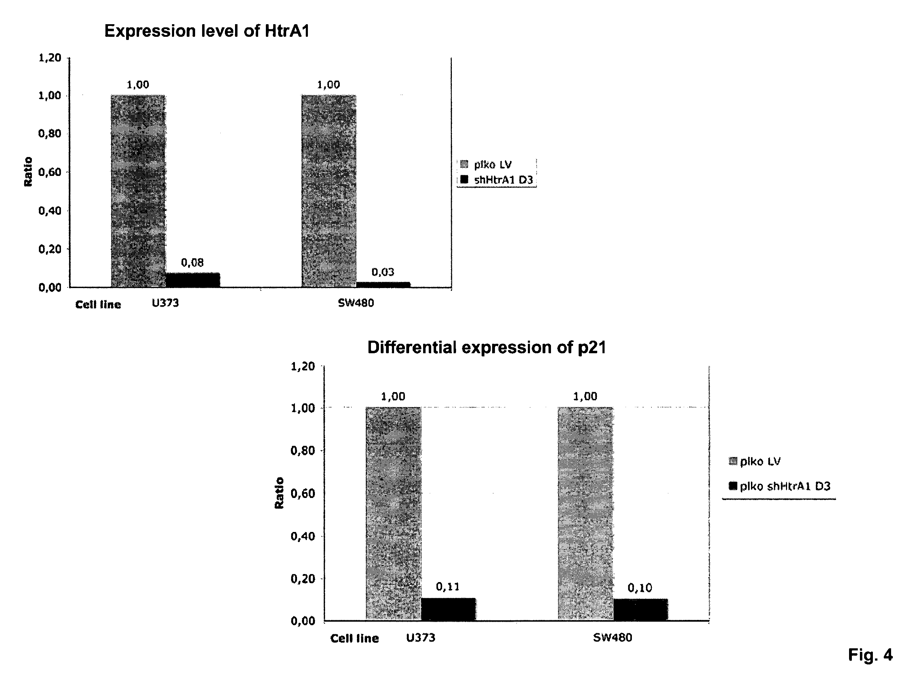

**Fig. 4****: The absence of HtrA1 correlates with the absence of p21** Analysis of HtrA1 and p21 expression in U373 (astrocytoma) and SW480 (colon cancer) cells transduced with an shRNA against HtrA1 or an empty vector control. The HtrA1-and p21-RNA level were analysed by quantitative RT-PCR. HtrA1 level was significantly downregulated (more than 90%) in U373 and SW480 cells expressing the shRNA against HtrA1 compared to the empty vector control. P21 level was also significantly downregulated (90%) in both cell lines expressing the shRNA against HtrA1.

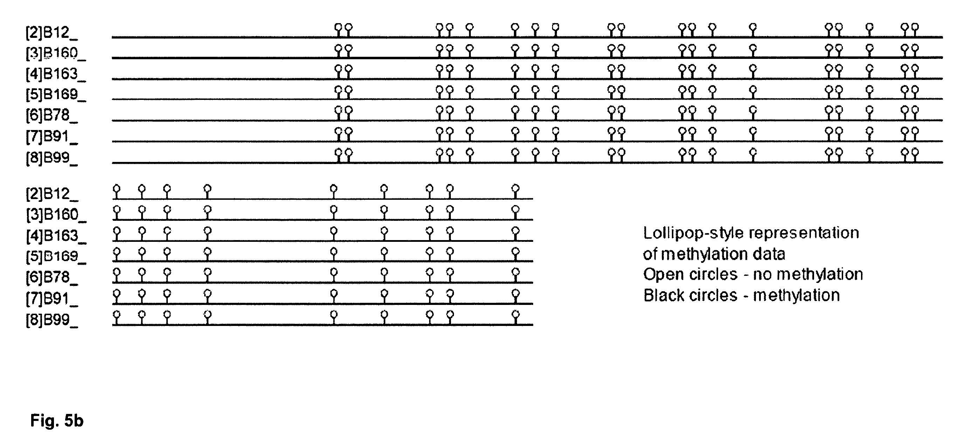

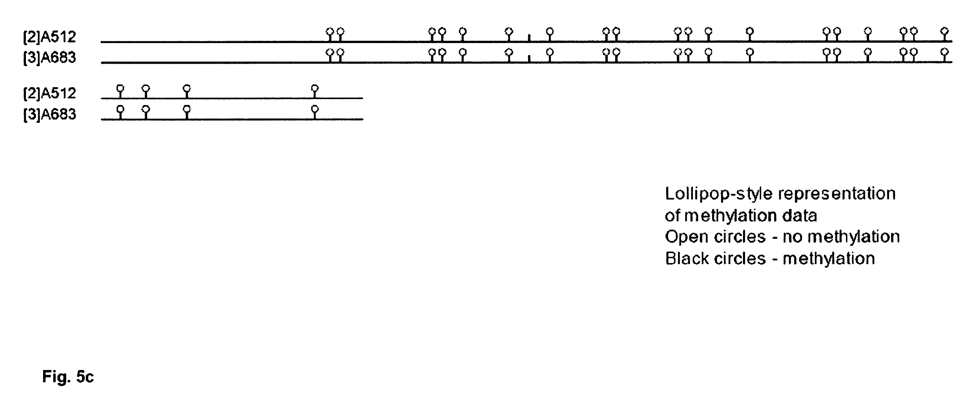

**Fig 5****: The expression of HtrA1 is epigenetically controlled in cancer but not in non-cancer cells (DNA-methylation of the HtrA1 promoter).** DNA-methylation level of the HtrA1 promoter was analyzed after bisulfite treatment of the DNA samples followed by bisulfite specific PCR using primers corresponding to a part of the CpG-island located in the promoter region of HtrA1. Filled (black) circles correspond to methylated Cs, unfilled (white) circles correspond to unmethylated Cs, and small vertical lines without a circle correspond to non-CpG positions in the genomic sequence.
   A) The graphic shows the correlation of DNA-Methylation and HtrA1 expression level of the HtrA1 promoter in SW480, HCT116 and 293T cancer cell lines. HtrA1 expression was measured by quantitative real-time PCR.
   B) DNA-Methylation of the HtrA1 promoter in human brain samples of 7 different patients is presented. As can be seen, no methylation occurs in brain tissue/samples.
   C) DNA-Methylation of the HtrA1 promoter in blood samples of 2 patients. As can be seen, no methylation occurs in blood cells/samples.
**Fig 6****: The expression of HtrA1 is epigenetically controlled in cancer cells** Expression of HtrA1 in HCT116 and SW480 cells treated with 5-Aza-dC and TSA. Cells were treated with 5-Aza-dC and TSA or solvent only. RNA was extracted from the cells followed by reverse transcription. cDNA was analyzed by quantitative real-time PCR.
**Fig 7****: The epigenetic factor MBD2 regulates HtrA1 by direct interaction with the HtrA1 promoter** MBD2 binds to HtrA1 and regulates HtrA1 transcription:
   **A)** Relative HtrA1 und MBD2 expression was measured by quantitative real-time PCR in HCT116 cells with knocked down MBD2 and control cells.
   **B)** ChIP enrichment of MBD2 at the HtrA1 promoter (Primer -4373 bp and -941 bp flanking a region upstream of the CpG-island and Primer -785 bp flanking a region within the CpG-island of the HtrA1 promoter) and a region between GAPDH and CNAP1 gene serving as a negative control. The enrichment was measured using quantitative real-time PCR, normalized by input DNA in HCT116 parental cells (left bars), HCT116 shMBD2 cells (right bars) and HCT116 vector control cells (middle bars).
**Fig 8****: Section of the HtrA1 gene**
   Shown is a section of the HtrA1 gene, namely the sequence 1048 base pairs upstream of ATG plus 1052 bp downstream of the ATG codon. Further introns and exons follow the shown coding sequence of the HtrA1 gene.
   The lower cases identify the 3' UTR; namely from -1048 bp to -1 bp.
   The upper cases identify at least part of the CDS of HtrA1; namely from +1 bp to +1052 bp.
   The bold cases identify at least a portion of the CpG-island; namely from - 588 bp to + 614 bp.
   The region shaded in grey in Fig. 8 identifies the MBD2 binding site as presently identified by CHIP; namely from - 785 bp to - 667 bp. This region corresponds to Figure 7b.
   The underlined region symbolises the overall region of the HtrA1 promotor analyzed for the methylation pattern/status, corresponding to Figure 5; region -762 bp to -383 bp.

### II. Detailed description of practicable examples and experiments

The present invention and its background is described by the following non-limiting examples.

### Example 1: Levels of HtrA1 during the cell cycle

**A) Cell culture**
   The cell line SW480 derived from human colon adenocarcinoma (DMSZ no. ACC 313) and the cell line U373 MG derived from human glioblastoma astrocytoma (ECACC no. 89081403) were cultured in DMEM (Gibco, Invitrogen) supplemented with 10% fetal bovine serum (FBS) and 1 % penicillin-streptomycin at 5% CO₂ and 37°C. The media for the stable transduced SW480 cell lines additionally contain 1.8 µg/ml puromycin.
**B) Cell cycle synchronisation with aphidicolin**
   5x10⁵ cells/ well were seeded into a six well plate. After 8 h, cells were treated with 1.25 µg/ml aphidicolin (Calbiochem, #178273). After 16 h, cells were washed three times with DMEM containing 10% FBS and 1% penicillin-streptomycin. Then cells were allowed to grow for 8 h. This treatment was repeated two times. 6 h and 9 h after the last wash cell were harvested and fixed.
**C) Cell cycle analysis by flow cytometry (FACS)**
   Cells were harvested by treatment with trypsin/EDTA. Cells were centrifuged for 5 minutes at 1700 g and 4°C. The pellet was resuspended and washed in 1 ml PBS. Cells were centrifuged again for 5 minutes at 1700 g and 4°C. The supernatant was discarded and the pellet was resuspended in 100 µl PBS. After addition of 500 µl ice-cold methanol (-80°C) cells were incubated at -20°C for 60 minutes and then centrifuged for 5 minutes at 1700 g and 4°C. The pellet was resuspended in 500 µl PBS containing 0.1% Triton X-100 and incubated at room temperature for 10 minutes. After addition of 5 µl RNase A (10 mg/ml) and 15 µl propidium iodide (2 mg/ml, Sigma #P4170), cells were incubated in the dark at 4°C for 60 minutes and then analysed at the flow cytometer (FACScalibur, BD biosciences)
**D) Simultaneous cell cycle analysis and intracellular immunofluorescence at the flow cytometer**
   Cells were harvested by treatment with trypsin/EDTA. Cells were centrifuged for 5 minutes at 1700 g and 4°C. The pellet was resuspended and washed in 1 ml PBS. Cells were centrifuged again for 5 minutes at 1700 g and 4°C. The supernatant was discarded and the pellet was resuspended in 100 µl PBS. After addition of 500 µl ice-cold methanol (-80°C) cells were incubated at -20°C for 60 minutes and then centrifuged for 5 minutes at 1700 g and 4°C. The pellet was resuspended in 500 µl PBS containing 0.1% Triton X-100 and incubated at room temperature for 10 minutes. Cells were washed with 1 ml PBS containing 2% FBS (5 min, 1700 g, 4°C). Pellets were resuspended in 100 µl PBS containing 2% FBS and polyclonal HtrA1-antibody (1:50) and incubated at room temperature for 30 minutes. Cells were washed two times in 1 ml PBS containing 2% FBS (5 min, 1700 g, 4°C). Pellets were resuspended in 100 µl PBS containing 2% FBS and FITC-conjugated second antibody (goat-anti-rabbit; 1:500) and incubated in the dark for 30 minutes at 4°C. Cells were washed two times in 1 ml PBS containing 2% FBS (5 min, 1700 g, 4°C). After addition of 5 µl RNaseA (10 mg/ml) and 15 µl propidium iodide (2 mg/ml, Sigma #P4170) cells were incubated in the dark at 4°C for 60 minutes and then analysed at the flow cytometer (FACScalibur, BD biosciences)

### Example 2: Karyotyping of SW480 cells

Exponentially growing cultures (*SW480 plko LV and SW480 plko shHtrA1 D3)* were incubated in N-deacetyl-N-Methylcolchicine (Colcemid; 0.08 mg/ml) for 2 h in order to arrest mitotic cells in highly condensed metaphase like stages. Monolayers were then rinsed and the cells were collected in centrifuge tubes. Following a 5 min centrifugation at 1200 g cell sediments were hypotonically treated with 5 ml of 75 mM potassium chloride for 10 min. Following centrifugation the swollen cells were gently mixed with 5 ml of fixing solution (methanol/acetic acid; 3/1), centrifuged, and again mixed with fixing solution.

Cell suspensions were dropped onto pre-cleaned, wet, ice-cold glass microscope slides to obtain good spreading of the chromosome sets. After an overnight air-drying the preparations were stained in Giemsa-solution (5%). Intact metaphase cells (those that were not disrupted during the preparation step) were counted for their chromosome numbers at 1000 fold magnification (oil-immersion).

### Example 3: HtrA1 activity is inhibited by Cytochalasin E

Enzymatic activity of HtrA1 was assayed using acyl p-nitroanilide (p-NA) substrate FANQHLCGSHLVEA-p-NA. HtrA1 (5 µg) was preincubated with Cytochalasin E (25 µM) for 10 minutes at 37°C. Reaction was initiated by adding p-NA substrate at a final concentration of 0.5 mM. The volume of each reaction was 100 µl in 50 mM Tris-HCl pH 8. Reaction was measured continuously in a spectrophotometer for 70 minutes at 37°C at 405 nm.

### Example 4: RNA extraction and qPCR

4 x 10⁵ SW480 cells/well were seeded into a six-well plate. After two days the six-well plate was 80% confluent and RNA was extracted by using total RNA isolation kit (Macherey-Nagel). RNA was purified with NucleoSpin RNAII columns (Macherey-Nagel) according to the procedure by the manufacturer. 1 µg of total RNA was used for cDNA synthesis by Verso^{™} cDNA kit (Thermo Scientific); the cDNA synthesis was performed as described by the manufacturer.

Quantitative RT-PCR was done with Platinum SYBR Green qPCR-superMix-UDG-kit (Invitrogen) with Rotor-Gene RG3000 from Corbett Research. The following oligonucleotides were used:
Human-HtrA1-forward-GCAACTCAGACATGGACTACATC;
Human-HtrA1-reverse-GTGTTAATTCCAATCACTTCACCG;
Human-p21/CDKN1a-forward-CCTCATCCCGTGTTCTCCTTT;
Human-p21/CDKN1a-reverse-GTACCACCCAGCGGACAAGT;
Human-GAPDH-forward-GCTTGTCATCAATGGAAATCCC;
Human-GAPDH-reverse-AGCCTTCTCCATGGTGG;
Human-RibProtL13a-forward-GGTGGTCGTACGCTGTG;
Human-RibProtL13a-reverse-GGTCCGCCAGAAGATGC.

The total reaction volume was of 25 µl, containing 12.5 µl SYBR Green PCR Master Mix (Invitrogen), 1 µl Primer (4.5 µM each) and 4 µl cDNA (20 ng). The following cycling conditions were used, 95°C for 15 minutes, followed by 40 cycles of denaturation at 95°C for 15 s, annealing at 55°C for 30 s and elongation at 72°C for 30 s. Melting curve was measured every 5 seconds from 50°C to 99°C. Gene expression ratios for each sample were calculated as described (Pfaffl, Nucleic Acids Res, 29:e45 (2001)). Melting curve analysis confirmed that only one product was amplified.

### Example 5: DNA-methylation level of the HtrA1 promoter

**A) Bisulfite modification of genomic DNA** Bisulfite Treatment of DNA leads to conversion of all cytosine residues to uracil in unmethylated DNA. Methylated cytosines are not affected by this treatment.
   Genomic DNA was isolated from the cells using QiaAMP DNA Mini Kit (Qiagen) according to the manufacturer's protocol. 2 µg of isolated genomic DNA were used in the bisulfite modification reaction with the EpiTect Kit (Qiagen) as recommended by the manufacturer.
**B) Bisulfite PCR**
   2 µl bisulfite treated genomic DNA were used as a template for the HtrA1 promoter specific PCR. The amplification of the HtrA1 promoter region was performed in two rounds. In the first round a primer pair specific for the HtrA1 promoter was used:
   HtrA1 forward: GTTATTTATAATAATTTTTTAAAAGAATTAG;
   HtrA1 reverse: TCCTTCAAACTAATAAAACTTTACAAA.

The primers were designed using Methyl Primer express software (Applied Biosystems). 10 µl of the first PCR product were used in the second PCR. Primers used in this PCR were tagged to allow direct sequencing of the PCR product:
HtrA1 forward SP6: **ATTTAGGTGACACTATAGAA**GTTATTTATAATAATTTTTTAAAAGAATTAG
HtrA1 reverse T7:

### TTAATACGACTCACTATAGGGCCTTCAAACTAATAAAACTTTACAAA.

The PCR reactions were carried out in a total volume of 100 µl using 500 nM of each primer, 200 µM of each dNTP, 2.5 U Hot Star Taq DNA Polymerase (Qiagen) and the buffer supplied with the enzyme. The reaction mix was activated for 15 minutes at 95°C. The PCR amplifications were carried out as follows: 40x (94°C for 1 min, 50°C for 1 min, 68°C for 1 min) and 68°C for 10 min for the first PCR and 5x (94°C for 1 min, 50°C for 1 min, 68°C for 1 min) 35x (94°C for 1 min, 58°C for 1 min, 68°C for 1 min) and 68°C for 10 min for the second PCR round.

The PCR-product was loaded on a 1,5% agarose gel and extracted from agarose gel using QIAquick Gel Extraction Kit (Qiagen) according to manufacturer's instructions. The PCR products were directly sequenced. Sequencing results were evaluated using BiQ-Analyzer.

### Example 6: Effect of 5-Aza-2'-deoxycytidine and Trichostatin A on HtrA1 expression

**A) Treatment of cells with the DNA methylation inhibitor 5-Aza-2'-deoxycytidine (5-Aza-dC) and the HDAC inhibitor Trichostatin A (TSA)** Cells were seeded at a density of 5 x 10⁵/ 60 mm dish and allowed to attach over 24 h in a 37°C incubator in a humified atmosphere with 5% CO2. 5-aza-2'-deoxycytidine was solved in 50% acetic acid. It was added to the medium for a final concentration ranging from 0 to 10 µM for 72 h. At every 24 h interval, fresh medium containing the drug was added. Cells were incubated in a 37°C incubator in a humified atmosphere with 5 % CO2.
   For the study of synergistic effects of 5-aza-2'-deoxycytidine and TSA, cells were first incubated with 5 µM and 10 µM 5-aza-2'deoxycytidine for 56 h and then 400 nM TSA was added for an additional 16 h incubation in a 37 °C incubator in a humified atmosphere with 5% CO₂.
   At the end of the treatment cells were washed twice with PBS and RNA was isolated for analysis of HtrA1 expression using quantitative Real-Time-PCR.
**B) RNA isolation** RNA was isolated using NucleoSpin-RNA-Clean-Up-Kit (Macherey-Nagel) according to the manufacturer's instructions.
**C) Reverse Transcription** cDNA synthesis was performed using Reverse-IT-1st Strand Synthesis Kit (ABGene) according to the manufacturer's protocol. The reaction was carried out at a total volume of 20 µl. 1 µg of purified RNA was used for cDNA-synthesis.
**D) Quantitative Real-Time PCR** Quantitative Real-Time PCR was performed on the RotorGene3000 (Corbett Life Science) using Absolute QPCR SYBR Green Mix (ABgene) according to the manufacturer's instructions. 1 µl of cDNA, 12.5 µl Absolute QPCR SYBR Green Mix and 400 nM of each primer were used for the quantitative real-time PCR reaction. The reaction mix was activated at 95 °C for 15 min. PCR amplification was done for 40 cycles at 95 °C for 15 s, 56 °C for 30s, 72 °C for 30 s.

The relative gene expression level of HtrA1 was calculated relative to the GAPDH and PPIA genes according to the method of Pfaffl (Pfaffl Nucleic Acids Res 29:e45 (2001)).

Primer sequences for measuring gene expression levels:
HtrA1 forward: GCAACTCAGACATGGACTACATC;
HtrA1 reverse: GTGTTAATTCCAATCACTTCACCG;
GAPDH forward: GCTTGTCATCAATGGAAATCCC;
GAPDH reverse: AGCCTTCTCCATGGTGG
PPIA forward: CAAATGCTGGACCCAACACA
PPIA reverse: CTTGCTGGTCTTGCCATTCC
MBD2 forward: AACCCTGCTGTTTGGCTTAAC
MBD2 reverse: CGTACTTGCTGTACTCGCTCTTC

### Example 7: Knockdown of MBD2 and HtrA1 using RNAi-Technology and viral gene transfer

**A) MBD2 shRNA** For generating the shRNA vector, a 48 bp hairpin sequence directed against MBD2 was cloned into the lentiviral pLKO.1puro vector (restriction sites: *Agel* and *EcoRI*) containing puromycin resistance.
The hairpin sequence for shMBD2: sense: 
CCGGGGAAGTGATCCGAAAATCTTTCTCGAGAAAGATTTTCGGATCACTTCCTTTT
TG (bold/black: AgeI restriction site, bold/underlined: loop)
 antisense: 
AATTAAAAAGGAAGTGATCCGAAAATCTTTCTCGAGAAAGATTTTCGGATCACTTC
C (bold/black: EcoRI restriction site, bold/blue: loop)
 **HtrA1 shRNA**
The hairpin sequence for shHtrA1: sense: 
CCGGGATCTCAGGAGCGTATATATTCTCGAGAATATATACGCTCCTGAGATCTTTT
TG
 antisense:

CTAGAGTCCTCGCATATATAAGAGCTCTTATATATGCGAGGACTCTAGAAAAACTT
AA

**Hybridisation reaction**

| |
|---|
| 20 µl T4-DNA-Ligase buffer (Invitrogen) |
| 75 µl H₂O |
| 2.5 µl sense Oligo (100 µM) |
| 2.5 µl antisense Oligo (100 µM) |

**Hybridisation program**

| | |
|---|---|
| 99°C | 1 min |
| 96°C | 7 min |
| 85°C | 5 min |
| 75°C | 7 min |
| 65°C | 10 min |
| 37°C | 10 min |
| 22°C | 20 min |
| 16°C | 10 min |
| 4°C | HOLD |

The DNA-Hybrid and the vector were then cut by *EcoRI* and *Agel* and joined by ligation. Positive clones were identified by colony-PCR after heat shock transformation in electro competent *E. coli* cells.
**B) Gene transfer using viral vector systems** Viral vectors were developed to transfer a target gene into a cell. Retro- and Lentiviral derived vectors are especially eligible for the development of transport vectors, since they integrate into the genome of the infected cell in a stable manner.
Vectors used for gene transfer are replication deficient and thus undergo only one viral replication cycle. Due to this vector design, virus particles infect the target cell, integrate into the genome of the cell after reverse transcription and allow the expression of the target gene. Another replication cycle and spreading of the virus to other cells is not possible. Viral vectors are therefore useful tools for gene transfer, since they allow the stable transfer of a gene into cells without the need to expose them to replicating viruses.
To establish a replication deficient vector, the gag-, pol-, and env coding regions are eliminated from the genome of the virus and replaced by the gene of interest. Merely the regions for the detection of the viral and cellular proteins during the different stages of the viral replication cycle remain within the genome.
In order to produce infectious virus, components for packaging and enzymatic reactions must be provided. This is achieved by co-transfection of co-vectors that code for gag, pol and env. The co-transfection allows production and packaging of virions. These virions are then used for transduction of the target cells. The co-vectors lack the components necessary for replication and packaging and are therefore not transferred to the target cells. Thus the virus undergoes only one replication cycle and the target gene can be transferred in a directed and controlled manner.
To avoid recombination of the viral vector with the co-vectors that leads to production of a wildtype-virus, the split genome strategy was developed. The target gene, gag, pol and env are coded by three separate vectors to minimize the likelihood of production of a replication competent virus.
**C) Lentiviral Transduction Day 1:** 293T cells were seeded at a density of 3 x 10⁶ per 10 cm dish and allowed to attach over night in a 37°C incubator in a humified atmosphere with 10% CO₂.
**Day 2:** The calcium-phosphate precipitate was prepared by mixing 12 µg of pCMVΔR8.2 (contains gag and pol), 6 µg of pHITG (contains env), and 12 µg of lentiviral transfer vector containing the desired shRNA sequence and H₂O to a final volume of 438 µl. A lentiviral vector containing the GFP gene and lacking the antibiotics resistance gene is also transfected as a control. 62 µl 2 M CaCl₂-solution were added and mixed by pipetting. Thereafter 500 µl 2x HBS-Buffer were added dropwise and incubated for 10 min at room temperature. The mixture was added dropwise to the medium of the 293T-cells and incubated for 16 h in a 37°C incubator in a humified atmosphere with 10 % CO2.
**Day 3:** Medium from the 293T cells was removed and new medium was added. Cells were incubated in a 32°C incubator in a humified atmosphere with 5% CO₂. Transfection efficiency was examined by the means of the GFP-transfected 293T cells.
For each transduction 2 x 10⁵ target cells were seeded on 6-well plates and allowed to attach overnight in a 37°C incubator in a humified atmosphere with 10% CO₂.
**Day 4:** The virus-containing supernatant of the 293T cells was removed and sterile filtrated. Fresh medium was added to the 293 T cells and cells were incubated in a 32°C incubator in a humified atmosphere with 5% CO₂ overnight. 4 µg/ml Polybrene was added to the virus containing supernatant. Medium was removed from the target cells and cells were washed twice with PBS. Afterwards 2,5 ml of virus-containing supernatant were added to the target cells. Cell were incubated in a 32°C incubator in a humified atmosphere with 5% CO₂ overnight.
**Day 5:** The virus-containing supernatant of the 293T cells was removed and sterile filtrated. 293T cells were disposed. 4 µg/ml Polybrene was added to the virus containing supernatant. Medium was removed from the target cells and cells were washed twice with PBS.
Subsequently 2.5 ml of virus-containing supernatant were added to the target cells. Cell were incubated in a 37 °C incubator in a humified atmosphere with 10 % CO2 for 72 h.
**Day 8:** Transfection efficiency was examined by the means of the GFP-transfected target cells.
Medium of the target cells was changed and cells were allowed to recover in a 37°C incubator in a humified atmosphere with 10% CO₂ for 24 h.
**Day 9:** Puromycine is added in the appropriate concentration to the cells to start selection and assure stable expression of the transferred construct. Puromycine is also added to the GFP-transfected control cell line that does not contain the antibiotic resistance gene to control cell death. Stable expression of the transferred construct was evaluated by quantitative Real-Time-PCR, Western-Blot analysis and indirect immunofluorescence.

### Example 8: Chromatin Immunoprecipitation (CHIP)

The binding of MBD2 to the HtrA1 promoter was analysed by CHIP as follows:
Cells are grown on a 14.5 cm dish until 90% confluence and harvested by adding Trypsin-EDTA and 3 minutes incubation in a 37°C incubator. Complete medium containing serum is added to the Trypsin-EDTA cell-solution to terminate the proteolytic reaction. Then the cells are pelleted by centrifugation at 1200 rpm for 5 minutes. The supernatant is removed and the cells are resuspended in 7.2 ml PBS. The cells are crosslinked by adding formaldehyde to a final concentration of 1% while stirring. The cells are incubated for 10 minutes on a roller incubator at 4°C. To terminate the crosslink reaction glycine is added to a final concentration of 0.125 mM and the cells are incubated for 10 minutes in a roller-incubator at 4°C. Subsequently, the cells are pelleted by centrifugation (1000 rpm, 4 minutes) and the supernatant is discarded.
The cell pellet is washed twice with 5 ml ice cold PBS. Afterwards the cells are washed 3 times with 5 ml lysis buffer containing protease inhibitors. Cells are pelleted by centrifugation (1000 rpm, 4°C) between washes. The supernatant is discarded. The cells can be flash-frozen and stored at -80°C.
The cell pellet is resuspended in 1.5 ml pre-IP dilution buffer containing protease inhibitors.
Then the cells are sonicated to shear the DNA to fragments of a final average size of 100-500 bp. Afterwards the sonicated samples are centrifuged at 13200 rpm at 4°C to remove cellular debris. 700 µl of supernatant are transferred to a 1.5 ml tube. 60 µl of Salmon Sperm Protein-A Agarose beads are added to pre-clear the chromatin by incubation on rotating platform for 1 hour at 4°C. The beads are pelleted by centrifugation (1500 rpm, 3 minutes, 4°C) and the supernatant is transferred to a new tube. 3 µg of anti-MBD2 antibody (Upstate, 07-198) is added to the supernatant. A no antibody control is also performed using the same amount of chromatin. The samples are incubated over night at 4°C on a rotating platform. 80 µl Salmon-Sperm Protein A agarose beads are added to the samples and incubated for 2 hours at 4 °C on rotating platform. The beads are pelleted by centrifugation (1500 rpm, 3 minutes, 4°C) and the supernatant is discarded. The pellet is resuspended in 700 µl CHIP wash 1, transferred to Spin-X columns and incubated at room temperature for 1 minute. The column is centrifuged (1500 rpm, 1 minute, 4°C) and beads are washed once in 700 µl ChIP wash 2, once in 700 µl CHIP wash 3 and twice in 700 µl TE-buffer. The flow through is discarded after each wash step. Then the beads are resuspended with 100 µl elution buffer and incubated at 65°C for 30 minutes. The columns are centrifuged (3000 rpm, 2 minutes, room temperature) to collect the enriched immunoprecipitated sample.

To reverse the crosslinks 10 µl proteinase K (10 mg/ml), 4 µl 5 M NaCl and 5 µl RNAseA are added per 100 µl sample and the samples are incubated at 65°C overnight. At this step the samples can be stored at -20°C.

The DNA is purified using the Qiagen PCR purification Kit. The DNA is analyzed using quantitative real-time PCR.

The following primers were used in order to amplify different sections of the HtrA1 promoter. Thereby it was identified, to which region/part of the HtrA1 promoter MBD2 was cross-linked. Results are shown in Fig. 7b.
ChIP_HtrA1_-4373
Forward: TTTCTTGCCCTCCTTTCTC
Reverse: TAATCTCTATCTGTGCTGTCC
ChIP_HtrA1_-941 bp
Forward: GCACCAAAGATTCTCTCCAGT
Reverse: GGTCTCAGATGGGAAAGGGG
ChIP_HtrA1_-785 bp
Forward: TCTCTGCGAATACGGACAC
Reverse: AGACCCACAGTGAAGTGAT
ChIP_HtrA1_-413 bp
forward: CCCCTTGCAAAGTTCCATTA
reverse: CTCTCGCGGGACTCAGTTTC
ChIP_negative control
Forward: ATGGTTGCCACTGGGGATCT
Reverse: TGCCAAAGCCTAGGGGAAGA

### III. Results

The above described examples allow the following conclusions:

### A change in HtrA1 levels is detected during the cell cycle

Synchronised colon adenocarcinoma SW480 cells change the levels of HtrA1 during the cell cycle. Example 1 identified that cells in G2/M-phase have the highest level of HtrA1 and cells in G1-phase of the cell cycle have the lowest level of HtrA1. These results suggest that HtrA1 participates in the cell cycle or in the control of the cell cycle. Please also refer to Fig. 1.

### The absence of HfrA1 causes polyploidy

If HtrA1 plays an important role in the cell cycle, its downregulation or complete absence should produce a relevant phenotype. Therefore, Example 7 identified SW480 cells that were depleted for HtrA1 by using stable shRNA constructs. Example 1 identified, by using FACS analysis, a strong increase in polyploidy. The main peaks observed are 4n and 8n instead of 2n and a small peak of 4n in control cells. In addition, the longer the cells are cultured, the higher the polyploidy. Example 2 provided additional and direct evidence for polyploidy via karyotyping.

The polyploidy results are also shown in Figs. 2a and 2b.

### Cytochalasin E inhibits HfrA1 on the enzymatic level

It is known from the literature that cytochalasin B causes polyploidy by inhibition of actin polymerisation and that high amounts of cytochalasin B induce p21 and cell cycle arrest (Ganem et al. Cell 131:437-40 (2007);Baatout et al. Anticancer Res 18:459-64 (1998)). The fact that cytochalasin E efficiently inhibits HtrA1 links HtrA1 function to the cell cycle and the control of polyploidy (Example 3). It can be envisaged that HtrA1 plays a role in the homeostasis of the actin cytoskeleton and thus the absence of HtrA1 alters the cytoskeleton resulting in polyploidy. The results of the Htra1 inhibition by cytochalasin E (CytE) are also shown in Fig. 3.

The finding that cytochalasin E inhibits the serine protease HtrA1 is novel as cytochalasins have so far only been described as inhibitors of HIV protease which is a cysteine protease (e.g. U.S. Pat. No. 5192668, WO/1996/039142 and Lingham et al. Biochem Biophys Res Commun 181:1456-61 (1991); Dombrowski et al. J of Antibiot 45:671 (1992); Ondeyka et al. J Antibiot 45:679-685 (1992); Lingham, et al. J of Antibiot 45:686 (1992)). It should be noted, however, that cytochalasin E has not previously described to inhibit proteases.

### The absence of HtrA1 causes the absence of p21

Surprisingly, the polyploid cells proliferated as well as cells that were not depleted for HtrA1 (data not shown). This implicates that the cell cycle checkpoint that would put a halt on proliferation of polyploid cells is non-functional in HtrA1 depleted cells. The most likely candidate for the cell cycle checkpoint affected is the tumour suppressor p21 as it is known to be involved in the response to polyploidy (Ganem et al. Cell 131:437-40 (2007)). Indeed, example 4 indicates that p21 mRNA levels are downregulated by a factor of 10 when HtrA1 is expressed at much lower levels (as was achieved by expression of shRNA against HtrA1). This event correlated with the complete absence of p21 protein as determined by Western blotting (data not shown). These results explain why polyploid cells do not enter cell cycle arrest, a phenomenon that is critical for cancer cells.

The correlation of HtrA 1 expression and p21 expression is also shown in Fig. 4.

### Epigenetic control of HtrA1 expression in cancer but not in non-cancer cells

It has been described that HtrA1 expression is low in many samples of human cancer and it was suggested that, therefore, HtrA1 might function as a tumour suppressor (Chien et al. Oncogene 23:1636-44 (2004); (Chien et al. J Clin Invest 116:1994-2004 (2006)). The underlying mechanism for the lower expression of HtrA1 in cancer is, however, unknown and may have several causes. The analysis of the methylation of a CpG island in the HtrA1 promoter (example 5) revealed that HtrA1 expression is epigenetically controlled in cancer cells to various degrees. In contrast, no DNA methylation in the CpG island of the HtrA1 promoter has been detected in human brain and blood samples. Therefore, this finding provides the mechanism by which HtrA1 is downregulated in many cancer cells. The entire CpG island apparently spans the region comprising base pairs -588 and +614 of the HtrA1 gene (see Fig. 8).

The results are also shown in Figs. 5a to 5c.

Furthermore, example 6 indicates that the application of 5-Aza-dC and TSA, inhibitors of DNA methyltransferases and histone deactylases, respectively, to cancer cells lead to a large increase in HtrA1 expression, adding further support for the model of epigenetic control. The increase of HtrA 1 expression is believed to be due to an increase in histone acetylation.

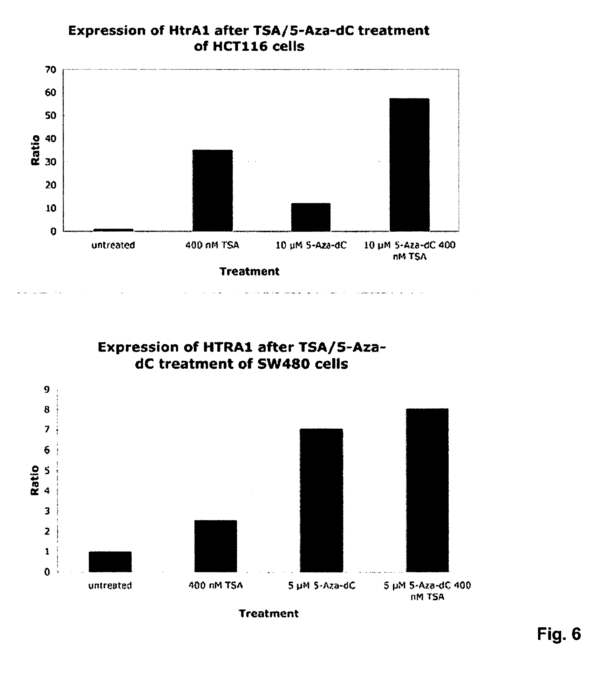

The results are also shown in Fig. 6.

### The epigenetic factor MBD2 regulates HtrA1

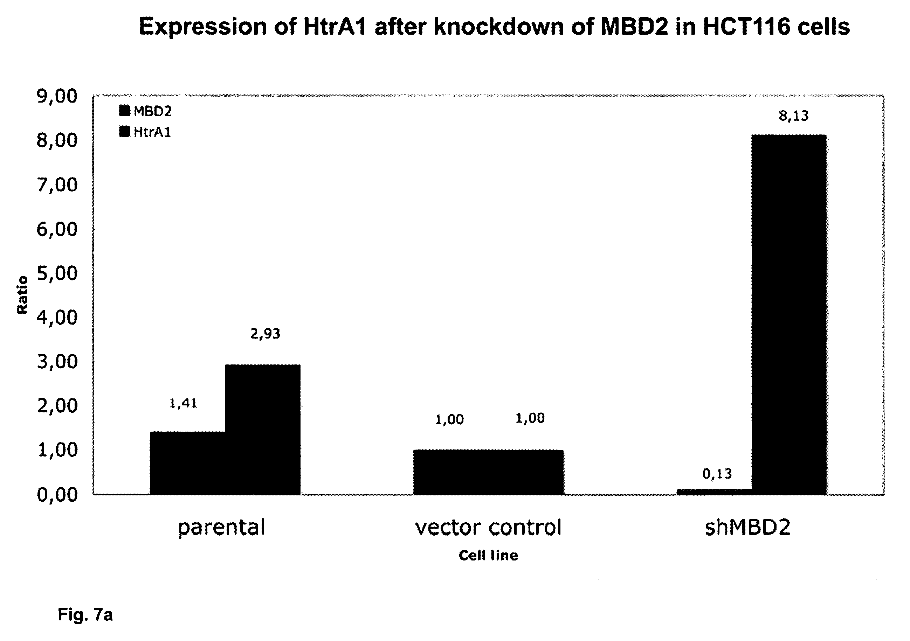

MBD2 binds to methylated DNA and recruits HDACs for histone deacetylation, a mechanism that switches promoters off. Example 7 indicates that MBD2 depleted cells respond with increased HtrA1 expression suggesting that MBD2 is involved in the epigenetic control of HtrA1 in cancer cells. This model is supported by CHIP experiments (see example 8) providing evidence for the direct interaction of MBD2 with a specific region of the HtrA1 promoter. These results suggest that the downregulation of HtrA1 in human cancer cells is mediated by epigenetic factors involving DMTAs, MBD2 and HDACs. These findings are in line with the important role of MBD2 in tumourigenesis (Sansom et al. Nat Genet. 34:145-7 (2003)).
The figures are shown in Figs. 7a and 7b.

### Methods for screening DNA methylation of or near the CpG island in HfrA1

Methods are therefore provided by the present invention for identifying a human subject having an increased risk of developing cancer or of acquiring the disease at an earlier age or for being sensitive or resistant to chemotherapy. The methods comprise identifying a nucleic acid sample from the subject that is methylated in the HtrA1 promoter. A specific region that was identified as relevant comprises an approximately 117 bp region around bp -785 in the HtrA1 promoter. The same might be true for single nucleotide polymorphisms located in the CpG island of the HtrA1 promoter. These SNPs can affect epigenetic control by altering the DNA sequence. Specifically, the screening method comprises detection of the level of methylation of the CpG island in HtrA1 for example by PCR amplification, bisulfite treatment and subsequent DNA sequencing as described in Example 5. As the level of DNA methylation correlates with the level of downregulation of HtrA1 expression, the results of this or related screening methods would not only detect cancerous cells and how aggressive this cancer is or will be but would also provide predictive information on if a cancer diagnosed in a given subject is sensitive or resistant to classical chemotherapies (Chien et al. J Clin Invest 116:1994-2004 (2006)).

### Methods for therapeutic intervention

The identification of the HtrA1 protein as an important player in the control of polyploidy and the control of cell cycle arrest as well as its regulation by epigenetic factors, especially by MBD2, points directly to a novel approach for therapeutic intervention that focuses on the restoration of HtrA1 expression to normal levels in cancer cells. This therapy is based on activation of HtrA1 on either the transcriptional or the enzymatic levels. Transcriptional activation can be brought about for example by inhibiting MBD2, HDACs and DMTAs. These factors can be either downregulated by inhibiting transcription factors regulating their own promoters or by small molecules directly interfering with their biological activities. These biological activities can be enzymatic or the interaction between the target proteins with its substrate; for example the interaction of DTMAs with unmethylated DNA, HDACs with acetylated histones or MBD2 and methylated DNA. In cases where the expression of HtrA1 is not completely shut off, enzymatic upregulation of HtrA1 can be accomplished by using activating compounds that bind to the PDZ domains of HtrA1 or other regions of the protein that are involved in the regulation of protease activity. Such compounds can be peptides or derivatives of peptides, natural compounds and their derivatives as well as chemical compounds.

### References

Hanahan D, Weinberg RA. The hallmarks of cancer. Cell 2000 100:57-70. Ganem NJ, Storchova Z, Pellman D.Tetraploidy, aneuploidy and cancer. Curr Opin Genet Dev 2007 17:157-62.

Harris SL, Levine AJ. The p53 pathway: positive and negative feedback loops Oncogene 2005 24:2899-2908.

Aylon Y, Oren M. Living with p53, dying of p53. Cell 2007 130:597-600.

Brito DA, Rieder CL. Mitotic checkpoint slippage in humans occurs via cyclinB destruction in the presence of an active checkpoint. Curr Biol 2006 16:1194-1200.

Ganem NJ, Pellman D Limiting the proliferation of polyploid cells. Cell 2007 131:437-40

Bestor TH. Gene silencing. Methylation meets acetylation. Nature 1998 393:311-2.

Bird, A.P. and Wolffe, A.P. Methylation-induced repression - Belts, braces, and chromatin. Cell 1999 99:451-454

Ting AH, McGarvey KM, Baylin SB. The cancer epigenome-components and functional correlates. Genes Dev 2006 20:3215-31.

Gallinari P, Di Marco S, Jones P, Pallaoro M, Steinkühler C. HDACs, histone deacetylation and gene transcription: from molecular biology to cancer therapeutics. Cell Res 2007 17:195-211.

Gore SD, Baylin S, Sugar E, Carraway H, Miller CB, Carducci M, Grever M, Galm O, Dauses T, Karp JE, Rudek MA, Zhao M, Smith BD, Manning J, Jiemjit A, Dover G, Mays A, Zwiebel J, Murgo A, Weng LJ, Herman JG. Combined DNA methyltransferase and histone deacetylase inhibition in the treatment of myeloid neoplasms. Cancer Res 2006 66:6361-9

Abou-Sleiman PM, Muqit MM, Wood NW. Expanding insights of mitochondrial dysfunction in Parkinson's disease. Nat Rev Neurosci 2006; 7:207-19.

Clausen T, Southan C, Ehrmann M. The HtrA family of proteases: implications for protein composition and cell fate. Mol Cell 2002; 10:443-55.

Grau S, Baldi A, Bussani R, Tian X, Stefanescu R, Przybylski M, Richards P, Jones SA, Shridhar V, Clausen T, Ehrmann M. Implications of the serine protease HtrA1 in amyloid precursor protein processing. Proc Natl Acad Sci USA 2005 102:6021-6

Nie G, Li Y, He H, Findlay JK, Salamonsen LA. HtrA3, a serine protease possessing an IGF-binding domain, is selectively expressed at the maternal-fetal interface during placentation in the mouse. Placenta 2006 27:491-501

Zumbrunn J, Trueb B. Primary structure of a putative serine protease specific for IGF-binding proteins. FEBS Lett 1996 398:187-92.

Shridhar V, Sen A, Chien J, Staub J, Avula R, Kovats S, Lee J, Lillie J, Smith DI. Identification of underexpressed genes in early- and late-stage primary ovarian tumours by suppression subtraction hybridization. Cancer Res 2002 62:262-70.

Chien J, Staub J, Hu SI, Erickson-Johnson MR, Couch FJ, Smith DI, Crowl RM, Kaufmann SH, Shridhar V. A candidate tumour suppressor HtrA1 is downregulated in ovarian cancer. Oncogene 2004 23:1636-44.

Baldi A, De Luca A, Morini M, Battista T, Felsani A, Baldi F, Catricala C, Amantea A, Noonan DM, Albini A, Natali PG, Lombardi D, Paggi MG. The HtrA1 serine protease is down-regulated during human melanoma progression and represses growth of metastatic melanoma cells. Oncogene 2002 21:6684-8.

Bowden MA, Di Nezza-Cossens LA, Jobling T, Salamonsen LA, Nie G. Serine proteases HTRA1 and HTRA3 are down-regulated with increasing grades of human endometrial cancer. Gynecol Oncol 2006 103:253-60.

Grau S, Richards PJ, Kerr B, Hughes C, Caterson B, Williams AS, Junker U, Jones SA, Clausen T, Ehrmann M. The role of human HtrA1 in arthritic disease. J Biol Chem. 2006 281:6124-9.

Chien J, Aletti G, Baldi A, Catalano V, Muretto P, Keeney GL, Kalli KR, Staub J, Ehrmann M, Cliby WA, Lee YK, Bible KC, Hartmann LC, Kaufmann SH, Shridhar V. Serine protease HtrA1 modulates chemotherapy-induced cytotoxicity. J Clin Invest. 2006 116:1994-2004.

Dewan A, Liu M, Hartman S, Zhang SS, Liu DT, Zhao C, Tam PO, Chan WM, Lam DS, Snyder M, Barnstable C, Pang CP, Hoh J. HTRA1 promoter polymorphism in wet age-related macular degeneration. Science 2006 314:989-92.

Yang Z, Camp NJ, Sun H, Tong Z, Gibbs D, Cameron DJ, Chen H, Zhao Y, Pearson E, Li X, Chien J, Dewan A, Harmon J, Bernstein PS, Shridhar V, Zabriskie NA, Hoh J, Howes K, Zhang K. A variant of the HTRA1 gene increases susceptibility to age-related macular degeneration. Science 2006 314:992-3.

Lingham RB, Arison BH, Colwell LF, Hsu A, Dezeny G, Thompson WJ, Garrity GM, Gagliardi MM, Hartner FW, Darke PL, et al. HIV-1 protease inhibitory activity of L-694,746, a novel metabolite of L-689,502. Biochem Biophys Res Commun 1991 181:1456-61.

Dombrowski, et al., L-696,474, A Novel Cytochalasin as an Inhibitor of HIV-1 Protease 1. The Producing Organism and its Fermentation. J of Antibiot 1992 45:671.

Ondeyka J, Hensens OD, Zink D, Ball R, Lingham RB, Bills G, Dombrowski A, Goetz M. L-696,474, a novel cytochalasin as an inhibitor of HIV-1 protease II. Isolation and structure. J Antibiot 1992 45:679-685 Lingham, et al., L-696,474, A Novel Cytochalasin as an Inhibitor of HIV-1 Protease III. Biological Activity. J of Antibiot 1992 45:686

Baatout S, Chatelain B, Staquet P, Symann M, Chatelain C. Inhibition of actin polymerization by cytochalasin B induces polyploidization and increases the number of nucleolar organizer regions in human megakaryocyte cell lines. Anticancer Res 1998 18:459-64.

Zupkovitz G, Tischler J, Posch M, Sadzak I, Ramsauer K, Egger G, Grausenburger R, Schweifer N, Chiocca S, Decker T, Seiser C. Negative and positive regulation of gene expression by mouse histone deacetylase 1. Mol Cell Biol. 2006 26:7913-28

Sansom OJ, Berger J, Bishop SM, Hendrich B, Bird A, Clarke AR. Deficiency of Mbd2 suppresses intestinal tumourigenesis. Nat Genet 2003 34:145-7.

Pfaffl MW. A new mathematical model for relative quantification in real-time RT-PCR. Nucleic Acids Res 2001 29:e45

## Claims

1. A method for analysing a biological sample, wherein the epigenetic status of at least one section of the HtrA 1 gene is analysed.

2. A method according to claim 1, wherein the epigenetic status of at least one section of the HtrA 1 promoter is analysed.

3. A method according to claim 1 or 2, wherein said epigenetic status is analysed by analysing the
- DNA methylation status,
- histone acetylation status, and/or
- expression and/or binding of an epigenetic factor.

4. The method according to one of the claims 1 to 3, wherein the methylation status of at least one CpG island in least one section of the HtrA 1 promoter as shown in Fig. 8 is analysed.

5. The method according to one of the claims 2 to 4, wherein said at least one section of the HtrA1 promoter comprises or consists at least a portion of a sequence selected from the group consisting of
a. the sequence shown in Fig. 8;
b. the sequence 
gccacccacaacaacttttaaaagaatcagacgtgtgaaggattctatt
cgaattacttctgctctctgcttttatcacttcactgtgggtctgggcgc
gggctttctgccagctccgcggacgctgccttcgtccggccgcagaggcc
ccgcggtcagggtcccgcgtgcgggtaccgggggcagaaccagcgcgtg
accggggtccgcggtgccgcaacgccccgggtctgcgcagaggcccctgc
agtccctgcccggcccagtccgagcttcccggcgggcccccagtccggc
gatttgcaggaactttccccggcgctcccacgcgaagccgccgcagggcc
cccttgcaaagttccattagtttgaagga;
c. the sequence 
cgcgaatctcagcgagagaacctgcggaaagcgaatatgtggggcgcgca
gacggggaaactgagtcccgcgagagggccggcctgtgcgctgccccgcc
cgcgccccgccagcaccgccgtgcccgggcgcgcccgccctgccccctcc
gcgggcggtcccggtccagccgcccgccctccctcccgccatccggccag
cccccatcccgggcgccgtgcccgtccccaaggcggctcgtcaccgctgc
 
gaggccaatgggctgggccgcgcggccgcgcactcgcacccgctgccc
ccgaggccctcctgcactctccccggcgccgctctccggccctcgccctg
tccgccgccaccgccgccgccgccagagtcgcc;
d. the sequence 
atgcagatcccgcgcgccgctcttctcccgctgctgctgctgctggc
ggcgcccgcctcggcgcagctgtcccgggccggccgctcggcgcctttgg
ccgccgggtgcccagaccgctgcgagccggcgcgctgcccgccgcagccg
gagcactgcgagggcggccgggcccgggacgcgtgcggctgctgcgaggt
gtgcggcgcgcccgagggcgccgcgtgcggcctgcaggagggcccgtgcg
gcgaggggctgcagtgcgtggtgcccttcggggtgccagcctcggccacg
gtgcggcggcgcgcgcaggccggcctctgtgtgtgcgccagcagcgagcc
ggtgtgcggcagcgacgccaacacctacgccaacctgtgccagctgcgcg
ccgccagccgccgctccgagaggctgcaccggccgccggtcatcgtcctg
cagcgcggagcctgcggccaaggtactccgccgcgctcctgggcagctcc
ccactctctccatcccagctcggacctgcttctgcgggactggtgggcag
gttgaggggcagcgaagcgttgtggggtggccagggcaactctcggggac
aggcaggtgggccc
 and/or a functional variant thereof.

6. The method according to one of the claims 1 to 5, wherein the acetylation status of the histones is analysed.

7. The method according to at least one of the claims 1 to 6, wherein the expression level
of an epigenetic factor binding the methylated and/or the unmethylated form of the HtrA 1 gene is analysed.

8. The method according to claim 7, wherein the expression level of the epigenetic factor MBD2 is analysed.

9. The method according to at least one of the claims 1 to 8, wherein the biological sample comprises or consists of tumour and/or cancer cells.

10. The method according to at least one of the claims 1 to 9, wherein nucleic acids are isolated from tumour and/or cancer tissue, and the methylation status of at least one section of the HtrA 1 promoter is analysed.

11. A method for analysing and/or diagnosing cancer and/or a tumour, **characterised in that** the method according to at least one of the claims 1 to 10 is performed.

12. A diagnostic kit for performing a method according to at least one of the claims 1 to 11.

13. The diagnostic kit according to claim 12 for analysing the methylation status of at least one section of the HtrA 1 gene, wherein said kit comprises primers for amplifying at least a portion of a sequence selected from the group consisting of
a. the sequence shown in Fig. 8;
b. the sequence 
gccacccacaacaactttttaaaagaatcagacgtgtgaaggattctatt
cgaattacttctgctctctgcttttatcacttcactgtgggtctgggcgc
gggctttctgccagctccgcggacgctgccttcgtccggccgcagaggcc
ccgcggtcagggtcccgcgtgcggggtaccgggggcagaaccagcgcgtg
accggggtccgcggtgccgcaacgccccgggtctgcgcagaggcccctgc
agtccctgcccggcccagtccgagcttcccgggcgggcccccagtccggc
gatttgcaggaactttccccggcgctcccacgcgaagccgccgcagggcc
cccttgcaaagttccattagtttgaagga;
c. the sequence 
cgcgaatctcagcgagagaacctgcggaaagcgaatatgtggggcgcgca
gacggggaaactgagtcccgcgagagggccggcctgtgcgctgccccgcc
cgcgccccgccagcaccgccgtgcccgggcgcgcccgccctgcccctcc
gcgggcggtcccggtccagccgcccgccctccctcccgccatccggccag
cccccatcccgggcgccgtgcccgtccccaaggcggctcgtcaccgctgc
gaggccaatgggctgggccgcgcggccgcgcgcactcgcacccgctgccc
ccgaggccctcctgcactctccccggcgccgctctccggccctcgccctg
tccgccgccaccgccgccgccgccagagtcgcc;
d. the sequence

atgcagatcccgcgcgccgctcttctcccgctgctgctgctgctggc
ggcgcccgcctcggcgcagctgtcccgggccggccgctcggcgcctttgg
ccgccgggtgcccagaccgctgcgagccggcgcgctgcccgccgcagccg
gagcactgcgagggcggccgggcccgggacgcgtgcggctgctgcgaggt
gtgcggcgcgcccgagggcgccgcgtgcggcctgcaggagggcccgtgcg
gcgaggggctgcagtgcgtggtgcccttcggggtgccagcctcggccacg
gtgcggcggcgcgcgcaggccggcctctgtgtgtgcgccagcagcgagcc
ggtgtgcggcagcgacgccaacacctacgccaacctgtgccagctgcgcg
ccgccagccgccgctccgagaggctgcaccggccgccggtcatcgtcctg
cagcgcggagcctgcggccaaggtactccgccgcgctcctgggcagctcc
ccactctctccatcccagctcggacctgcttctgcgggactggtgggcag
gttgaggggcagcgaagcgttgtggggtggccagggcaactctcggggac

aggcaggtgggccc.

14. A screening method for identifying a molecule which inhibits the binding of an epigenetic factor to at least one section of the HtrA1 gene, comprising
a.) contacting an inhibitor candidate molecule with a sample comprising at least the epigenetic factor and a polynucleotide molecule comprising or consisting of the section of the HtrA 1 gene the epigenetic factor binds to,
b.) determining the degree of binding of the epigenetic factor to said polynucleotide.

15. Screening method according to claim 14, wherein said epigenetic factor is MBD2.
